# EUROPEAN PATENT APPLICATION

(11) **EP 4 553 840 A2**
(43) Date of publication of application: **14.05.2025**
(21) Application number: 25154524.0
(22) Date of filing: 15.03.2016
(51) Int. Cl.: G16B 30/20

(54) **SYSTEMS AND METHODS FOR ANALYZING NUCLEIC ACID**

(30) Priority: 16.03.2015 US 201562133638 P
(62) Divisional of application: 16765585.1
(71) Applicant: Personal Genome Diagnostics, Inc., Baltimore, MD 21224 (US)
(72) Inventor: VELCULESCU, Victor, Baltimore, 21036 (US); DIAZ, Luis, Ellicott City, 21043 (US); JONES, Siân, Baltimore, 21224 (US); ANGIUOLI, Samuel, Vincent, Ellicott City, 21042 (US)
(74) Representative: Mewburn Ellis LLP

(57) **Abstract**

Increased sensitivity and specificity of characterizing patient-specific variations as mutations that are indicative of a cancer or other disease by identifying patient-specific tumor mutations by comparing tumor and normal sequence reads from the patient and filtering for mutations that are unique to the tumor. By comparing tumor sequence to a normal sequence from the
same patient, false-positive mutation calls are minimized in the analysis.

## Description

### Cross-Reference to Related Application

This application claims the benefit of, and priority to, U.S. Provisional Patent Application No. 62/133,638, filed March 16, 2015, the contents of which are incorporated by reference.

### Field of the Invention

The invention relates to the analyzing nucleic acid for tumor-specific biomarkers.

### Background

Genomic analysis has become an integral part of healthcare. The accumulation of genomic mutations over time can be indicative of the presence, type and severity of disease. A thorough understanding of an individual's mutation profile can lead to personalized diagnostics, more accurate prognoses, and tailored treatment options that are useful to prolong the patient's life and help avoid painful and expensive treatments.

Personalized medicine is, in large part, dependent on accurate identification of mutations in a patient's genome, including DNA and RNA. While there are many diseases that can be typed and tracked with genomic screening, cancer mutation screening has received the most attention. In most instances, cancer screening involves obtaining a cancerous sequence from the patient (e.g., from the tumor tissue) and comparing the cancerous sequence to a reference sequence. The reference sequence is a representative sequence assembled from sequencing and compiling nucleic acid from a number of donors. The reference sequence can be obtained from a healthy, normal population of donors or from donors having a specific disease. In order to identify sequence variations, a putative cancer sequence may be compared to the normal reference, and differences between the two are indicative of sequence variations.

In some instances, sequence variations are useful as disease markers, as in the case of BRCA1 mutations and breast cancer. However, simply identifying sequence variation in the cancer is not effective and may result in false positives because every individual is unique and may have germline sequence variations from the normal reference that are not indicative of a tumor-specific mutation. In addition, other identified sequence variations may be the result of sequencing artifacts and other sequencing errors. In some cases, these sequencing errors can be indistinguishable from actual mutations. Misidentification of sequence variations can negate many of the benefits understanding an individual's genome. For example, if a normal sequence variation is misinterpreted as a cancerous mutation, this can lead to misdiagnosis, an incorrect prognosis, or ineffective treatment. Alternatively, if an actual cancerous mutation is incorrectly dismissed as a sequencing error or as a normal variation, then the patient may miss otherwise promising treatment opportunities.

### Summary

The present invention generally relates to highly-sensitive and specific methods and systems for characterizing sequence variations as disease-causing mutations. Methods of the invention compare a patient's own sequence obtained from a putative cancerous tissue with normal sequences from the same patient in order to filter and eliminate sequencing artifacts associated with the patient's healthy DNA or RNA. After filtering, only portions of the genome that are inconsistent with normal sequence are assessed as cancer mutations. As a result, any normal patient-specific variations present in a tumor sequence are not misidentified as cancerous mutations when the tumor sequence is compared against a reference sequence during cancer screening.

According to certain aspects, methods of the invention involve identifying patient-specific tumor mutations by comparing tumor and normal sequence reads from the patient and filtering for mutations that are unique to a tumor. That comparison allows those variations associated with patient's normal sequence to be excluded from further analysis by concluding that they are not derived from loci underlying the cancer, and focuses the analysis on only variations that are particular to the patient's tumor. The variations that are specific to the patient's tumor may be classified as patient-specific biomarkers. In certain embodiments, the patient-specific biomarkers can be further characterized or classified by comparing the tumor-specific variations to a known tumor reference. As a result of the patient-specific tumor analysis, an individualized prognosis and treatment regimen is developed for the patient based on the particular biomarkers found in the patient.

Methods of the invention involve obtaining a tumor sequence read and a normal sequence read from a patient. In one preferred embodiment, the tumor sample is collected by isolating circulating tumor DNA (ctDNA) from blood plasma. Using ctDNA with the methods described herein allow for a variety of tumor markers to be screened with high accuracy without requiring an invasive biopsy or surgery. It also allows for broad analysis when the patient's affliction (i.e. cancer source) is unknown or the patient may be diagnosed with more than one condition. The tumor sample can also be obtained from a biopsy specimen or any other method known in the art. The normal sample can be any sample from the patient containing tissue believed to be tumor-free, such as lymphocytes, saliva, a buccal sample, or other unaffected tissue.

Systems and methods of the invention involve providing or generating sequencing reads of nucleic acid obtained from a patient. Any sequencing platform may be used to sequence nucleic acid from the patient in order to generate sequence reads. Suitable sequencing techniques include, for example, single molecule real-time sequencing, ion semiconductor sequencing, pyrosequencing, sequencing by synthesis, sequencing by ligation, and Sanger sequencing.

After the patient nucleic acid is sequenced, the tumor and normal reads are each then compiled into a consensus sequence. The consensus sequences may be generated by forming a contig with the obtained sequence reads or by aligning the sequencing reads to a reference. The tumor and normal consensus sequences may be formed by the same method or different method. After the consensus sequences are formed, the normal consensus sequence and consensus sequence are compared to identify variations.

After the tumor and normal sequences are compared, methods of the invention provide for filtering the tumor sequence in order to only focus on non-normal variations. In certain embodiments, a threshold is used to determine whether a portion of the tumor sequence should be classified as normal (and thus filtered out) or classified as a variant specific to the tumor. In certain embodiments, any variation in the tumor sequence as compared to the normal sequence is identified as a variant sequence specific to the tumor. In other embodiments, variants specific to the tumor are identified based on their similarity or dissimilarity to the normal reference. For example, portions of the tumor sequence may be classified as variant specific to the tumor because it is varies from to a corresponding segment of the normal sequence to a degree of 75%, 80%, 85%, 90%, 95%, 96%, 97%, 98%, 99%, 99.1%, 99.5%, 99.6%, 99.7%, 99.8%, 99.9%, etc. In another example, portions of the tumor sequence may be classified as normal because it is similar from to a corresponding segment of the normal sequence to a degree of 75%, 80%, 85%, 90%, 95%, 96%, 97%, 98%, 99%, 99.1%, 99.5%, 99.6%, 99.7%, 99.8%, 99.9%, etc. In certain embodiments, the threshold chosen is the same or different for different types of mutation. For example, the threshold for single nucleotide polymorphisms may be different from the threshold chosen for translocations.

After filtering, the resultant variant sequences that are tumor specific can be further analyzed. In certain embodiments, the tumor-specific variant sequence may be identified as tumor biomarkers specific to the patient. These biomarkers are particularly useful in determining the stage of the tumor, monitoring progression, and evaluating course of treatment. In further embodiments, the tumor-specific variant sequence are compared to a reference sequence, such as a known tumor reference, to assess whether the variant sequence include mutations or match mutations associated with known cancer.

These variants specific to the tumor, as identified in accordance with methods of the invention, can be monitored over time to see if they increase in number, which would indicate that the cancer is progressing, or if they decrease, which would be indicate that it is remitting. For example, a patient may have received an analysis of his whole exome to pinpoint locations of interest for a previously-uncharacterized cancer. That analysis would help doctors determine what type of cancer it is. As a later follow-up assay, the tumor DNA could be analyzed for only certain genes now known to be associated with his cancer. If more biomarkers are discovered, that would indicate the cancer is continuing to mutate and spread. This targeted follow-up assay would help verify if the patient's treatment is working or if the cancer is spreading.

The methods disclosed herein provide comprehensive analyses for detection and interpretation of somatic and germline alterations in human cancer. The methods can identify alterations in tumors that may be clinically actionable. The methods can recognize, in apparently sporadic cancer patients, pathogenic germline changes in cancer predisposing genes.

### Brief Description of the Drawings

FIG. 1 shows a method of assessing for a tumor biomarker.
FIG. 2 shows genes of biological and clinical importance in human cancer.
FIG. 3 shows genes for which structural variations tend to indicate disease.
FIG. 4 diagrams a system of the invention.
FIG. 5 diagrams whole exome or targeted next generation sequencing analyses.
FIG. 6 shows cases with evidence for clinical actionability by tissue type.
FIG. 7 shows somatic alterations and germline false positive in a targeted analyses
FIG. 8 shows somatic alterations and germline false positive changes in exome analyses.
FIG. 9 summarizes characteristics and the number of somatic and germline variants.
FIG. 10 shows mutations of a targeted set of genes subject to COSMIC filtering.
FIG. 11 shows classification of mutations in the exome cases by the COSMIC criteria.
FIG. 12 shows targeted filtering for somatic mutations in tumor suppressor genes.
FIG. 13 shows filtering for somatic mutations in the exome cases.
FIG. 14 shows targeted filtering for mutations within a kinase domain.
FIG. 15 shows filtering for mutations within a kinase domain in the exome case.

### Detailed Description

The present invention generally relates to methods and systems for characterizing a patient's sequence variations as mutations indicative of a cancer or other disease with increased specificity and sensitivity. Methods of the invention involve using massively parallel sequencing approaches to characterize individual patient tumors and select therapies based on the identified mutations. Methods of the invention involve comparing a tumor sequence and normal sequence from a patient and filtering out the matching portions of the samples. The invention recognizes that accurate identification and clinical interpretation of alterations benefit from analysis of both tumor and normal DNA from cancer patients, and filtering them accordingly. The resulting filtered data only includes tumor-specific sequences (i.e. variants from the patient's tumor sequence). The tumor-specific variations may be indicative of the type, stage of cancer or progression of the cancer. In certain embodiments, the resultant tumor-specific variations are then compared to a reference sequence for further characterization. For example, the tumor-specific variations can be compared to a tumor reference sequence in order to identify the variations as known mutations associated with particular cancers. The tumor-specific biomarkers can also be compared to a normal reference.

High complexity genomic analyses are changing the diagnostic landscape of oncology. Therapies targeting specific genetic alterations can be safer and more effective than traditional chemotherapies when used in an appropriate patient population. That notion has been successfully demonstrated for a number of therapeutics targeting the protein products of specific genes that are altered in human cancer, including the use of imatinib in chronic myeloid leukemias carrying the BCR-ABL fusion, trastuzumab in ERBB2 (Her-2/neu) amplified breast cancer and vemurafenib in BRAF mutated melanoma. Molecular alterations have also been shown to have a predictive or prognostic effect. For example, mutations at codons 12 and 13 of KRAS predict a poor response to EGFR monoclonal antibodies such as cetuximab and panitumumab so the use of these drugs is contraindicated in colorectal cancer patients. Glioblastoma patients with IDH1-mutated tumors have an increased overall survival compared to those without such changes. In addition to established therapies, off-label indications and drugs in clinical trials can benefit from knowledge of alterations in specific genes. As the mutations driving each individual tumor are unique, identifying the specific mutations in each patient's cancer is critical for the development of a personalized treatment plan that takes advantage of the growing number of targeted therapies.

Each tumor contains inherited (germline) and tumor-specific (somatic) variants. Somatic alterations in oncogenes and tumor-suppressors drive the development and growth of the tumor and are typically the targets of personalized therapies. The present disclosure recognizes that sequencing and comparison of matched normal DNA to tumor DNA from an affected individual allows for accurate identification and subtraction of germline alterations from somatic changes. Most prior cancer diagnostic assays, including next generation sequencing approaches only assess tumor DNA, likely as a result of logistical difficulties in obtaining a blood or saliva sample, increased cost, and an under-appreciation of the potential value of the matched normal.

The present disclosure recognizes that accurate identification of clinically actionable tumor-specific (somatic) alterations is enhanced by analyzing normal DNA side by side with tumor DNA.

From a clinical perspective, the use of matched tumor and normal DNA for genomic analyses is the most direct approach for accurate identification of actionable somatic and germline changes in cancer specimens. Although hotspot mutations in a few oncogenes can be readily detected with high sensitivity and specificity by analyses of tumor tissue alone, up to a third of changes in targeted tumor-only analyses may be incorrectly classified as actionable somatic changes when these actually represent constitutional alterations. Use of additional bioinformatic filtering approaches can improve the specificity but will miss a sizable fraction of somatic changes in actionable genes. Additionally, without analysis of germline DNA, cancer patients cannot be accurately screened for hereditary mutations in cancer predisposition genes that could inform the clinical management of the patient and indicate additional family members that could benefit from regular cancer screening.

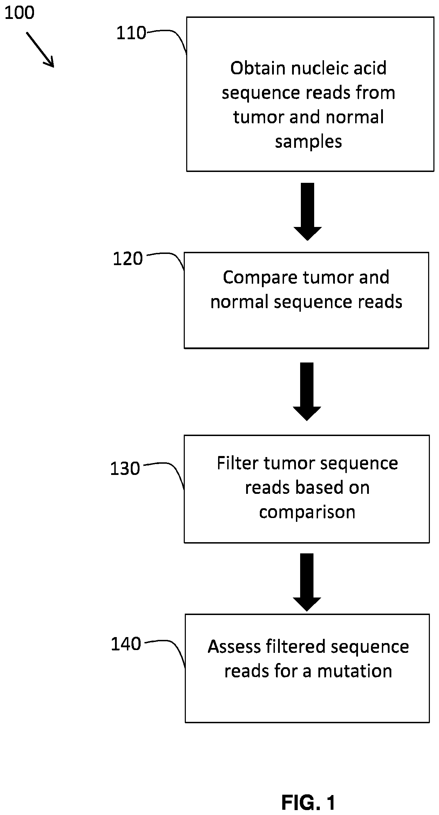

FIG. 1 shows a method 100 of assessing nucleic acid for a biomarker associated with a tumor. The method 100 begins with obtaining sequencing data from nucleic acid obtained from a tumor sample and a normal sample from the same patient in step 110. In certain embodiments, the tumor sample is a biopsy specimen, or from circulating tumor DNA (ctDNA). The normal sample can be any bodily tissue or fluid containing nucleic acid that is considered to be cancer-free, such as lymphocytes, saliva, buccal cells, or other tissues and fluids. The nucleic acids can be sequenced using any sequencing platform known in the art. The sequencing can be performed in conjunction with the invention, or a previously-obtained sequence read can be used.

After the tumor and normal sequence reads are obtained, they are compared to each other in step 120. In certain embodiments, the comparison involves forming a consensus sequence of the tumor and normal sequence reads, and then comparing the tumor consensus sequence to the normal consensus sequence. In certain embodiments, the consensus sequence (tumor, normal or both) is formed by generating a contig with the sequence reads. Alternatively, the consensus sequence (tumor, normal or both) is formed by aligning the sequence reads to a reference sequence. Any reference sequence can be used. In certain embodiments, the reference sequence is a representative sequence generated from a patient population, such as the human reference genome GRCh38 (the Genome Reference Consortium human genome (build 37)).

In step 130, the tumor sequence reads are filtered based on the comparison step 120. In certain embodiments, any variation in the tumor sequence as compared and filtered against the normal sequence is identified as a variant specific to the tumor. In other embodiments, variants specific to the tumor sequence are identified based on threshold that corresponds to a degree of similarity or dissimilarity to the normal reference. For example, portions of the tumor sequence may be classified as variant specific to the tumor because it is varies from to a corresponding segment of the normal sequence to a degree of 75%, 80%, 85%, 90%, 95%, 96%, 97%, 98%, 99%, 99.1%, 99.5%, 99.6%, 99.7%, 99.8%, 99.9%, etc. In another example, portions of the tumor sequence may be classified as normal because it is similar from to a corresponding segment of the normal sequence to a degree of 75%, 80%, 85%, 90%, 95%, 96%, 97%, 98%, 99%, 99.1%, 99.5%, 99.6%, 99.7%, 99.8%, 99.9%, etc. In certain embodiments, the threshold chosen is the same or different for different types of mutation. For example, the threshold for single nucleotide polymorphisms may be different from the threshold chosen for translocations.

After the tumor sequence reads are filtered, the filtered tumor sequence reads may be assessed in order to identify a mutation. In certain embodiments, the tumor-specific variant sequences (i.e. resultant tumor sequence after filtering) are identified as tumor biomarkers or mutations specific to the patient. In further embodiments, the tumor-specific variant sequences are compared to a reference, such as a known tumor reference, to assess whether the variant sequence include mutations or match mutations associated with known cancer.

Mutations identified and/or confirmed according to systems and methods of the invention may be used for disease screening in order to diagnose, monitor disease progression, and/or assess reoccurrence of disease. Methods and systems of the invention may be used to increase specificity and sensitivity in the identification of mutations in a variety of sequences and screening approaches. For example, applicable screening approaches may include screening of the patient's entire genome, entire exome, or targeted screens of specific genes or groups of genes. The vast majority of disease related mutations occur in the exome, or coding region of an individual's genetic material and therefore, screening the patient's exome according to systems and methods of the invention for a mutation associated with a condition may be more efficient than screening the entire genome.

In further embodiments, methods of the invention may target patient sequences known to relate to a disease or condition. For example, if the patient is known to have a particular condition, the screening may be limited to genes known to be associated with that condition. For example, if a tumor sample is obtained from a patient having lung cancer, then screening may be limited to genes associated with lung cancer.

In addition to lung cancer or leukemia, other genes or gene panels that are associated with one or more cancer types may be used for targeted screening of mutations. Those cancers may include, breast, skin, colorectal, pancreatic, ovarian, prostate, or cervical brain, cholangiocarcinomas, head and neck, neuroendocrine, renal, gastric, gynecological, esophageal, melanoma, hematopoietic malignancies, sarcomas, and many others. A list of genes known to be associated with a variety of cancers is provided in Table 1. Mutations in these known cancer associated genes can be used to diagnose, classify tumor subtypes, determine prognoses, monitor tumor progression, and establish appropriate therapies. Types of mutations identified using the systems and methods of the invention may include any type of mutation known in the art, including, for example, an insertion, a deletion, a copy number alteration, and/or a translocation.

| Table 1: List of known cancer associated genes for mutation screening | | | | | | | | | |
|---|---|---|---|---|---|---|---|---|---|
| *ABL1* | *AKT1* | *AKT2* | *ALK* | *APC* | *AR* | *ARID1A* | *ARID1B* | *ASXL1* | *ATM* |
| *ATRX* | *BAP1* | *BRAF* | *BRCA1* | *BRCA2* | *CBL* | *CCND1* | *CCNE1* | *CDH1* | *CDK4* |
| *CDK6* | *CDKN2A* | *CEBPA* | *CREBBP* | *CTNNB1* | *DAXX* | *DNMT3A* | *EGFR* | *ERBB2* | *ERBB3* |
| *ERBB4* | *EZH2* | *FBX27* | *FGFR2* | *FGFR3* | *FGFR4* | *FLT3* | *FOXL2* | *GATA1* | *GATA2* |
| *GNA11* | *GNAQ* | *GNAS* | *HNF1A* | *HRAS* | *IDH1* | *IDH2* | *IGFIR* | *IGF2R* | *IKZF1* |
| *JAK1* | *JAK2* | *JAK3* | *KDR* | *KITKRAS* | *MAML1* | *MDM2* | *MDM4* | *MED12* | *MEN1* |
| *MET* | *MLH1* | *MLL* | *MPL* | *MSH2* | *MSH6* | *MYC* | *MYCN* | *MYD88* | *NF1* |
| *NF2* | *NOTCH1* | *NOTCH2* | *NOTCH3* | *NOTCH4* | *NPM1* | *NRAS* | *PALB2* | *PAX5* | *PBRM1* |
| *PDGFRA* | *PDGFRB* | *PIK3CA* | *PIK3R1* | *PMS2* | *PTCH1* | *PTEN* | *PTPN11* | *RB1* | *RET* |
| *RNF43* | *ROS1* | *RUNX1* | *SF3B1* | *SMAD2* | *SMAD3* | *SMAD4* | *SMARCB1* | *SMO* | *STAG2* |
| *STK11* | *TET2* | *TGFBR2* | *TNFAIP3* | *TP53* | *TSC1* | *TSC2* | *TSHR* | *VHL* | *WT1* |

In certain embodiments, systems and methods of the invention may relate to a targeted analysis of the MET locus and surrounding regions in order to identify amplification of the MET gene. Amplification of the MET gene may trigger tumor growth and can be used for prediction of therapeutic response, overall prognosis, recurrence, monitoring, and early detection.

In certain embodiments, methods of the present disclosure are used to validate other bioinformatic approaches, such as approaches for separating somatic from germline mutations that rely only on tumor tissue, without the use of a matched normal.

The following describes the general methods for use with the invention as outlined in FIG. 1.

Systems and methods of the invention relate to obtaining sequencing data for a nucleic acid obtained from a patient. According to various embodiments, the nucleic acid may be from a tumor sample or a normal sample obtained from the patient. Cancer cells accumulate unique mutations from other, non-cancerous cells in a patient's body and often unique compared to other cancer cells of the same type from other individuals. Understanding the genetic sequence, including mutations, of a patient's cancer can help physicians provide more accurate diagnoses and prognoses and can inform targeted treatment decisions which may be more effective against certain genotypes of cancer. Accordingly, systems and methods of the invention may be applied to tumor sample sequencing. Understanding mutations in a patient's normal sample can be useful in understanding a patient's genetic predisposition to certain diseases and, therefore, implementation of a personalized screening regimen for early detection of those diseases in other family members. Furthermore, a patient's normal sequence along with the mutations therein, confirmed according to the systems and methods of the invention, may be used as a reference to screen a tumor sample sequence for tumor-specific mutations as described in more detail below.

Tumor samples may include, for example, cell-free nucleic acid (including DNA or RNA) or nucleic acid isolated from a tumor tissue sample such as biopsied tissue, formalin fixed paraffin embedded tissue (FFPE), frozen tissue, cell lines, DNA and tumorgrafts. Samples provided as FFPE blocks or frozen tissue may undergo pathological review to determine tumor cellularity. Tumors may be macrodissected or microdissected to remove contaminating normal tissue. Normal samples, in certain aspects, may include nucleic acid isolated from any non-tumor tissue of the patient, including, for example, patient lymphocytes, blood, saliva, cells obtained via buccal swab, or other unaffected tissue. Cell-free nucleic acids may be fragments of DNA or ribonucleic acid (RNA) which are present in the blood stream of a patient. In a preferred embodiment, the circulating cell-free nucleic acid is one or more fragments of DNA obtained from the plasma or serum of the patient. The cell-free nucleic acid may be isolated according to techniques known in the art and include, for example, the QIAmp system from Qiagen (Venlo, Netherlands), the Triton/Heat/Phenol protocol (THP) (Xue, et al., Optimizing the Yield and Utility of Circulating Cell-Free DNA from Plasma and Serum", Clin. Chim. Acta., 2009; 404(2): 100-104), blunt-end ligation-mediated whole genome amplification (BL-WGA) (Li, et al., "Whole Genome Amplification of Plasma-Circulating DNA Enables Expanded Screening for Allelic Imbalance in Plasma", J. Mol Diagn. 2006 Feb; 8(1): 22-30), or the NucleoSpin system from Macherey-Nagel, GmbH & Co. KG (Duren, Germany). In an exemplary embodiment, a blood sample is obtained from the patient and the plasma is isolated by centrifugation. The circulating cell-free nucleic acid may then be isolated by any of the techniques above.

According to certain embodiments, nucleic acid may be extracted from tumor or non-tumor patient tissues. Tumor DNA may be extracted, for example, from frozen or FFPE tissue, along with matched blood or saliva samples, using the Qiagen DNA FFPE tissue kit or Qiagen DNA blood mini kit (Qiagen, CA).

After tissue or cells have been obtained from the patient, it is often preferable to lyse or fragment cells in order to isolate nucleic acids. Lysing methods are known in the art. For example, lysing methods may include one or more of sonication, freezing, boiling, exposure to detergents, or exposure to alkali or acidic conditions. The concentration of the detergent can be up to an amount where the detergent remains soluble in the solution. The detergent, particularly one that is mild and nondenaturing, can act to solubilize the sample. Detergents may be ionic or nonionic. Examples of nonionic detergents include triton, such as the Triton^{®} X series (Triton^{®} X-100 t-Oct-C6H4-(OCH2-CH2)xOH, x=9-10, Triton^{®} X-100R, Triton^{®} X-114 x=7-8), octyl glucoside, polyoxyethylene(9)dodecyl ether, digitonin, IGEPAL^{®} CA630 octylphenyl polyethylene glycol, n-octyl-beta-D-glucopyranoside (betaOG), n-dodecyl-beta, Tween^{®} 20 polyethylene glycol sorbitan monolaurate, Tween^{®} 80 polyethylene glycol sorbitan monooleate, polidocanol, n-dodecyl beta-D-maltoside (DDM), NP-40 nonylphenyl polyethylene glycol, C12E8 (octaethylene glycol n-dodecyl monoether), hexaethyleneglycol mono-n-tetradecyl ether (C14EO6), octyl-beta-thioglucopyranoside (octyl thioglucoside, OTG), Emulgen, and polyoxyethylene 10 lauryl ether (C12E10). Examples of ionic detergents (anionic or cationic) include deoxycholate, sodium dodecyl sulfate (SDS), N-lauroylsarcosine, and cetyltrimethylammoniumbromide (CTAB). A zwitterionic reagent may also be used in the purification schemes of the present invention, such as Chaps, zwitterion 3-14, and 3-[(3-cholamidopropyl) dimethyl-ammonio]-1-propanesulfonate. It is contemplated also that urea may be added with or without another detergent or surfactant.

Lysis or homogenization solutions may further contain other agents, such as reducing agents. Examples of such reducing agents include dithiothretol (DTT), β-mercaptoethanol, DTE, GSH, cysteine, cystemine, tricarboxyethyl phosphine (TCEP), or salts of sulfurous acid.

By way of example, a lysing or fragmenting procedure may be performed with Illumina TruSeq library construction (Illumina, San Diego, CA) according to the manufacturer's instructions. For example, 50 nanograms (ng) to 3 micrograms (µg) of genomic DNA in 100 microliters (µl) of TE may be fragmented in a Covaris sonicator (Covaris, Woburn, MA) to a size of 150-450bp. To remove fragments smaller than 150bp, DNA can be purified using Agencourt AMPure XP beads (Beckman Coulter, IN) in a ratio of 1.0 to 0.9 of PCR product to beads twice and washed using 70% ethanol per the manufacturer's instructions.

Purified, fragmented DNA can be mixed with, for example, 36 µl of H2O, 10 µl of End Repair Reaction Buffer, 5 µl of End Repair Enzyme Mix (cat# E6050, NEB, Ipswich, MA). The 100 µl end-repair mixture can be incubated at 20°C for 30 min, and purified using Agencourt AMPure XP beads (Beckman Coulter, IN) in a ratio of 1.0 to 1.25 of PCR product to beads and washed using 70% ethanol per the manufacturer's instructions. To A-tail, 42 µl of end-repaired DNA can be mixed with 5 µl of 10X dA Tailing Reaction Buffer and 3 µl of Klenow (exo-)(cat# E6053, NEB, Ipswich, MA). The 50 µl mixture can be incubated at 37°C for 30 min and purified using Agencourt AMPure XP beads (Beckman Coulter, IN) in a ratio of 1.0 to 1.0 of PCR product to beads and washed using 70% ethanol per the manufacturer's instructions. For adaptor ligation, 25 µl of A-tailed DNA can be mixed with 6.7 µl of H2O, 3.3 µl of PE-adaptor (Illumina), 10 µl of 5X Ligation buffer and 5 µl of Quick T4 DNA ligase (cat# E6056, NEB, Ipswich, MA). The ligation mixture can be incubated at 20°C for 15 min and purified using Agencourt AMPure XP beads (Beckman Coulter, IN) in a ratio of 1.0 to 0.95 and 1.0 of PCR product to beads twice and washed using 70% ethanol per the manufacturer's instructions.

When there is an insufficient amount of nucleic acid for analysis, a common technique used to increase the amount by amplifying the nucleic acid. Amplification refers to production of additional copies of a nucleic acid sequence and is generally carried out using polymerase chain reaction or other technologies well known in the art (e.g., Dieffenbach and Dveksler, PCR Primer, a Laboratory Manual, 1995, Cold Spring Harbor Press, Plainview, NY).

By way of example, to obtain an amplified library, twelve PCRs of 25 µl each may be set up, each including 15.5 µl of H2O, 5 µl of 5 x Phusion HF buffer, 0.5 µl of a dNTP mix containing 10 mM of each dNTP, 1.25 µl of DMSO, 0.25 µl of Illumina PE primer #1, 0.25 µl of Illumina PE primer #2, 0.25 µl of Hotstart Phusion polymerase, and 2 µl of the DNA. A PCR program can be used, such as: 98°C for 2 minutes; 12 cycles of 98°C for 15 seconds, 65°C for 30 seconds, 72°C for 30 seconds; and 72°C for 5 min. DNA can be purified using Agencourt AMPure XP beads (Beckman Coulter, IN) in a ratio of 1.0 to 1.0 of PCR product to beads and washed using 70% ethanol per the manufacturer's instructions. Exonic or targeted regions can be captured in solution using the Agilent SureSelect v.4 kit or a custom targeted panel for the 111 genes of interest according to the manufacturer's instructions (Agilent, Santa Clara, CA). The captured library can then be purified with a Qiagen MinElute column purification kit and eluted in 17 µl of 70°C EB to obtain 15 µl of captured DNA library. The captured DNA library can be amplified in the following way: eight 30uL PCR reactions each containing 19 µl of H2O, 6 µl of 5 x Phusion HF buffer, 0.6 µl of 10 mM dNTP, 1.5 µl of DMSO, 0.30 µl of Illumina PE primer #1, 0.30µl of Illumina PE primer #2, 0.30 µl of Hotstart Phusion polymerase, and 2 µl of captured exome library can be set up. A PCR program can be used, such as: 98°C for 30 seconds; 14 cycles (exome) or 16 cycles (targeted) of 98°C for 10 seconds, 65°C for 30 seconds, 72°C for 30 seconds; and 72°C for 5 min. To purify PCR products, a NucleoSpin Extract II purification kit (Macherey-Nagel, PA) can be used following the manufacturer's instructions.

The amplification reaction may alternatively be any such reaction known in the art that amplifies nucleic acid molecules, including polymerase chain reaction, nested polymerase chain reaction, polymerase chain reaction-single strand conformation polymorphism, ligase chain reaction (Barany, F., Genome Research, 1:5-16 (1991); Barany, F., PNAS, 88:189-193 (1991); U.S. Pat. 5,869,252; and U.S. Pat. 6,100,099), strand displacement amplification and restriction fragments length polymorphism, transcription based amplification system, rolling circle amplification, and hyper-branched rolling circle amplification. Further examples of amplification techniques that can be used include, but are not limited to, quantitative PCR, quantitative fluorescent PCR (QF-PCR), multiplex fluorescent PCR (MF-PCR), real time PCR (RTPCR), single cell PCR, restriction fragment length polymorphism PCR (PCR-RFLP), RT-PCR-RFLP, hot start PCR, in situ polonony PCR, in situ rolling circle amplification (RCA), bridge PCR, picotiter PCR and emulsion PCR. Other suitable amplification methods include transcription amplification, self-sustained sequence replication, selective amplification of target polynucleotide sequences, consensus sequence primed polymerase chain reaction (CP-PCR), arbitrarily primed polymerase chain reaction (AP-PCR), degenerate oligonucleotide-primed PCR (DOP-PCR) and nucleic acid based sequence amplification (NABSA). Other amplification methods that can be used herein include those described in U.S. Pats. 5,242,794; 5,494,810; 4,988,617; and 6,582,938.

In certain embodiments, the amplification reaction is the polymerase chain reaction. Polymerase chain reaction (PCR) refers to methods by K. B. Mullis (U.S. Pats. 4,683,195 and 4,683,202, hereby incorporated by reference) for increasing concentration of a segment of a target sequence in a mixture of genomic DNA without cloning or purification.

Primers can be prepared by a variety of methods including but not limited to cloning of appropriate sequences and direct chemical synthesis using methods well known in the art (Narang et al., Methods Enzymol., 68:90 (1979); Brown et al., Methods Enzymol., 68:109 (1979)). Primers can also be obtained from commercial sources such as Operon Technologies, Amersham Pharmacia Biotech, Sigma, and Life Technologies. The primers can have an identical melting temperature. The lengths of the primers can be extended or shortened at the 5' end or the 3' end to produce primers with desired melting temperatures. Also, the annealing position of each primer pair can be designed such that the sequence and length of the primer pairs yield the desired melting temperature. The simplest equation for determining the melting temperature of primers smaller than 25 base pairs is the Wallace Rule (Td=2(A+T)+4(G+C)). Computer programs can also be used to design primers, including but not limited to Array Designer Software from Arrayit Corporation (Sunnyvale, CA), Oligonucleotide Probe Sequence Design Software for Genetic Analysis from Olympus Optical Co., Ltd. (Tokyo, Japan), NetPrimer, and DNAsis Max v3.0 from Hitachi Solutions America, Ltd. (South San Francisco, CA). The TM (melting or annealing temperature) of each primer is calculated using software programs such as OligoAnalyzer 3.1, available on the web site of Integrated DNA Technologies, Inc. (Coralville, IA).

Amplification adapters may be attached to the fragmented nucleic acid. Adapters may be commercially obtained, such as from Integrated DNA Technologies (Coralville, IA). In certain embodiments, the adapter sequences are attached to the template nucleic acid molecule with an enzyme. The enzyme may be a ligase or a polymerase. The ligase may be any enzyme capable of ligating an oligonucleotide (RNA or DNA) to the template nucleic acid molecule. Suitable ligases include T4 DNA ligase and T4 RNA ligase, available commercially from New England Biolabs (Ipswich, MA). Methods for using ligases are well known in the art. The polymerase may be any enzyme capable of adding nucleotides to the 3' and the 5' terminus of template nucleic acid molecules.

The ligation may be blunt ended or via use of complementary overhanging ends. In certain embodiments, following fragmentation, the ends of the fragments may be repaired, trimmed (e.g. using an exonuclease), or filled (e.g., using a polymerase and dNTPs) to form blunt ends. In some embodiments, end repair is performed to generate blunt end 5' phosphorylated nucleic acid ends using commercial kits, such as those available from Epicentre Biotechnologies (Madison, WI). Upon generating blunt ends, the ends may be treated with a polymerase and dATP to form a template independent addition to the 3'-end and the 5'-end of the fragments, thus producing a single A overhanging. This single A is used to guide ligation of fragments with a single T overhanging from the 5'-end in a method referred to as T-A cloning.

Alternatively, because the possible combination of overhangs left by the restriction enzymes are known after a restriction digestion, the ends may be left as-is, i.e., ragged ends. In certain embodiments double stranded oligonucleotides with complementary overhanging ends are used.

In certain embodiments, a single bar code is attached to each fragment. In other embodiments, a plurality of bar codes, e.g., two bar codes, are attached to each fragment.

After sufficient nucleic acid samples are obtained, they must be sequenced to determine which nucleic acid residues they contain, so that the normal and tumor sequences can be compared. There are various methods of sequencing known in the art, which are described in more detail below, including Sanger sequencing and various types of next generation sequencing.

Classical Sanger sequencing involves a single-stranded DNA template, a DNA primer, a DNA polymerase, radioactively or fluorescently labeled nucleotides, and modified nucleotides that terminate DNA strand elongation. If the label is not attached to the dideoxynucleotide terminator (e.g., labeled primer), or is a monochromatic label (e.g., radioisotope), then the DNA sample is divided into four separate sequencing reactions, containing four standard deoxynucleotides (dATP, dGTP, dCTP and dTTP) and the DNA polymerase. To each reaction is added only one of the four dideoxynucleotides (ddATP, ddGTP, ddCTP, or ddTTP). These dideoxynucleotides are the chain-terminating nucleotides, lacking a 3'-OH group required for the formation of a phosphodiester bond between two nucleotides during DNA strand elongation. If each of the dideoxynucleotides carries a different label, however, (e.g., 4 different fluorescent dyes), then all the sequencing reactions can be carried out together without the need for separate reactions.

Incorporation of a dideoxynucleotide into the nascent, i.e., elongating, DNA strand terminates DNA strand extension, resulting in a nested set of DNA fragments of varying length. Newly synthesized and labeled DNA fragments are denatured, and separated by size using gel electrophoresis on a denaturing polyacrylamide-urea gel capable of resolving single-base differences in chain length. If each of the four DNA synthesis reactions was labeled with the same, monochromatic label (e.g., radioisotope), then they are separated in one of four individual, adjacent lanes in the gel, in which each lane in the gel is designated according to the dideoxynucleotide used in the respective reaction, i.e., gel lanes A, T, G, C. If four different labels were utilized, then the reactions can be combined in a single lane on the gel. DNA bands are then visualized by autoradiography or fluorescence, and the DNA sequence can be directly read from the X-ray film or gel image.

The terminal nucleotide base is identified according to the dideoxynucleotide that was added in the reaction resulting in that band or its corresponding direct label. The relative positions of the different bands in the gel are then used to read (from shortest to longest) the DNA sequence as indicated. The Sanger sequencing process can be automated using a DNA sequencer, such as those commercially available from PerkinElmer, Beckman Coulter, Life Technologies, and others.

The other broad category of sequencing technologies is next generation sequencing, or NGS. Next-generation sequencing technologies provide low-cost high-throughput sequencing. Next generation typically produces a large number of independent reads, each representing anywhere between 10 to 1000 bases of the nucleic acid. Nucleic acids are generally sequenced redundantly for confidence, with replicates per unit area being referred to as the "coverage" (i.e., "10X coverage" or "100X coverage"). Thus, a multi-gene genetic screening can produce millions of reads.

Sequencing-by-synthesis is a common technique used in next generation procedures and works well with the instant invention. However, other sequencing methods can be used, including sequence-by-ligation, sequencing-by-hybridization, gel-based techniques and others. In general, sequencing involves hybridizing a primer to a template to form a template/primer duplex, contacting the duplex with a polymerase in the presence of a detectably-labeled nucleotides under conditions that permit the polymerase to add nucleotides to the primer in a template-dependent manner. Signal from the detectable label is then used to identify the incorporated base and the steps are sequentially repeated in order to determine the linear order of nucleotides in the template. Exemplary detectable labels include radiolabels, florescent labels, enzymatic labels, etc. In particular embodiments, the detectable label may be an optically detectable label, such as a fluorescent label. Exemplary fluorescent labels include cyanine, rhodamine, fluorescien, coumarin, BODIPY, alexa, or conjugated multi-dyes. Numerous techniques are known for detecting sequences and some are exemplified below. However, the exact means for detecting and compiling sequence data does not affect the function of the invention described herein.

In a preferred embodiment, nucleic acids are detected using single molecule sequencing. An example of a sequencing technology that can be used in the methods of the provided invention is Illumina sequencing. Illumina sequencing is based on the amplification of DNA on a solid surface using fold-back PCR and anchored primers. Genomic DNA is fragmented, and adapters are added to the 5' and 3' ends of the fragments. DNA fragments that are attached to the surface of flow cell channels are extended and bridge amplified. The fragments become double stranded, and the double stranded molecules are denatured. Multiple cycles of the solid-phase amplification followed by denaturation can create several million clusters of approximately 1,000 copies of single-stranded DNA molecules of the same template in each channel of the flow cell. Primers, DNA polymerase and four fluorophore-labeled, reversibly terminating nucleotides are used to perform sequential sequencing. After nucleotide incorporation, a laser is used to excite the fluorophores, and an image is captured and the identity of the first base is recorded. The 3' terminators and fluorophores from each incorporated base are removed and the incorporation, detection and identification steps are repeated.

Another example of a single molecule sequencing technique suitable for use in the methods of the provided invention is Ion Torrent sequencing (U.S. patent application numbers 2009/0026082, 2009/0127589, 2010/0035252, 2010/0137143, 2010/0188073, 2010/0197507, 2010/0282617, 2010/0300559), 2010/0300895, 2010/0301398, and 2010/0304982), the content of each of which is incorporated by reference herein in its entirety. In Ion Torrent sequencing, DNA is sheared into fragments of approximately 300-800 base pairs, and the fragments are blunt ended. Oligonucleotide adaptors are then ligated to the ends of the fragments. The adaptors serve as primers for amplification and sequencing of the fragments. The fragments can be attached to a surface and is attached at a resolution such that the fragments are individually resolvable. Addition of one or more nucleotides releases a proton (H+), which signal detected and recorded in a sequencing instrument. The signal strength is proportional to the number of nucleotides incorporated. User guides describe in detail the Ion Torrent protocol(s) that are suitable for use in methods of the invention, such as Life Technologies' literature entitled "Ion Sequencing Kit for User Guide v. 2.0" for use with their sequencing platform the Personal Genome MachineTM (PCG).

Another example of a DNA sequencing technique that can be used in the methods of the provided invention is 454 sequencing (Roche) (Margulies, M et al. 2005, Nature, 437, 376-380). 454 sequencing involves two steps. In the first step, DNA is sheared into fragments of approximately 300-800 base pairs, and the fragments are blunt ended. Oligonucleotide adaptors are then ligated to the ends of the fragments. The adaptors serve as primers for amplification and sequencing of the fragments. The fragments can be attached to DNA capture beads, e.g., streptavidin-coated beads using, e.g., Adaptor B, which contains 5'-biotin tag. The fragments attached to the beads are PCR amplified within droplets of an oil-water emulsion. The result is multiple copies of clonally amplified DNA fragments on each bead. In the second step, the beads are captured in wells (pico-liter sized). Pyrosequencing is performed on each DNA fragment in parallel. Addition of one or more nucleotides generates a light signal that is recorded by a CCD camera in a sequencing instrument. The signal strength is proportional to the number of nucleotides incorporated. Pyrosequencing makes use of pyrophosphate (PPi) which is released upon nucleotide addition. PPi is converted to ATP by ATP sulfurylase in the presence of adenosine 5' phosphosulfate. Luciferase uses ATP to convert luciferin to oxyluciferin, and this reaction generates light that is detected and analyzed.

Another example of a DNA sequencing technique that can be used in the methods of the provided invention is SOLiD technology (Applied Biosystems). In SOLiD sequencing, genomic DNA is sheared into fragments, and adaptors are attached to the 5' and 3' ends of the fragments to generate a fragment library. Alternatively, internal adaptors can be introduced by ligating adaptors to the 5' and 3' ends of the fragments, circularizing the fragments, digesting the circularized fragment to generate an internal adaptor, and attaching adaptors to the 5' and 3' ends of the resulting fragments to generate a mate-paired library. Next, clonal bead populations are prepared in microreactors containing beads, primers, template, and PCR components. Following PCR, the templates are denatured and beads are enriched to separate the beads with extended templates. Templates on the selected beads are subjected to a 3' modification that permits bonding to a glass slide. The sequence can be determined by sequential hybridization and ligation of partially random oligonucleotides with a central determined base (or pair of bases) that is identified by a specific fluorophore. After a color is recorded, the ligated oligonucleotide is cleaved and removed and the process is then repeated.

Another example of a sequencing technology that can be used in the methods of the provided invention includes the single molecule, real-time (SMRT) technology of Pacific Biosciences. In SMRT, each of the four DNA bases is attached to one of four different fluorescent dyes. These dyes are phospholinked. A single DNA polymerase is immobilized with a single molecule of template single stranded DNA at the bottom of a zero-mode waveguide (ZMW). A ZMW is a confinement structure which enables observation of incorporation of a single nucleotide by DNA polymerase against the background of fluorescent nucleotides that rapidly diffuse in an out of the ZMW (in microseconds). It takes several milliseconds to incorporate a nucleotide into a growing strand. During this time, the fluorescent label is excited and produces a fluorescent signal, and the fluorescent tag is cleaved off. Detection of the corresponding fluorescence of the dye indicates which base was incorporated. The process is repeated.

Another example of a sequencing technique that can be used in the methods of the provided invention is nanopore sequencing (Soni G V and Meller A. (2007) Clin Chem 53: 1996-2001). A nanopore is a small hole, of the order of 1 nanometer in diameter. Immersion of a nanopore in a conducting fluid and application of a potential across it results in a slight electrical current due to conduction of ions through the nanopore. The amount of current which flows is sensitive to the size of the nanopore. As a DNA molecule passes through a nanopore, each nucleotide on the DNA molecule obstructs the nanopore to a different degree. Thus, the change in the current passing through the nanopore as the DNA molecule passes through the nanopore represents a reading of the DNA sequence. Depending on what type of diagnostics need to be done, the whole genome may be sequenced, or just a specific part of particular interest.

In certain embodiments, the entire genome is sequenced for both the tumor sample and the normal sample. A whole-genome assay might be desirable where the patient has an unknown cancer and a broad approach is necessary to pinpoint the mutations present. When tumor nucleic acid is isolated from ctDNA, and the type or location of the tumor is otherwise unknown, it may be desirable to analyze the whole genome. The mutations in the ctDNA can potentially include mutations from many tumors in the body, so performing a broad analysis on ctDNA will give a more complete picture of the progression of cancer in the body.

For economic and efficiency reasons, it may be desirable to analyze just the exome. The exome is the coding region of the genome, and it comprises only about 1% of the entire genome. The exome is the target of most cancer mutations because these are the areas of the genome that are expressed. Isolating ctDNA and analyzing just the exome would still provide a broad picture of cancers present in the body, and would be easier and less expensive than sequencing a whole genome. The exome is a good place to start if sequencing the entire genome is prohibitively expensive or inefficient.

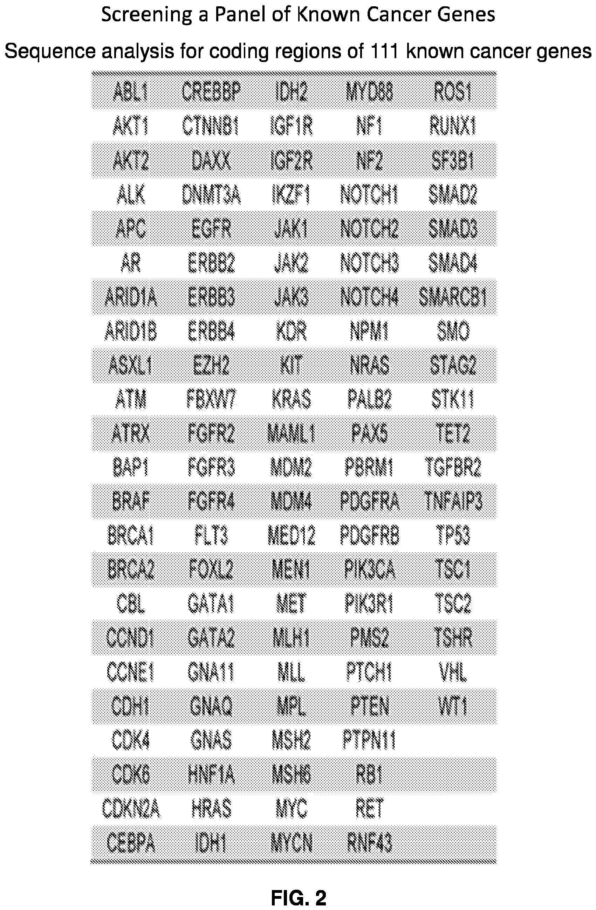

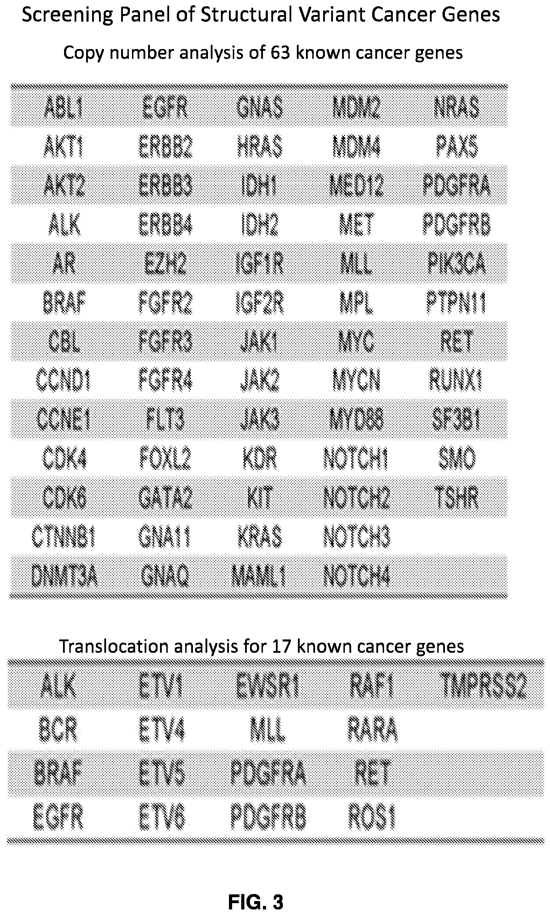

In another embodiment, a broad panel of known cancer-related genes may be assayed. FIGS. 2 and 3 shows various non-limiting examples of panels of known cancer genes and manners in which they may be screened.

FIG. 2 shows one hundred eleven genes of biological and clinical importance in human cancer, whose coding regions can be analyzed for mutations. Some of the types of cancer covered by this panel are breast cancer, colorectal cancer, leukemia, prostate cancer and lymphoma. Even though the number of genes sequenced in this assay has narrowed considerably from the whole-genome or whole-exome approaches, it still covers a broad range of human cancers.

FIG. 3 shows genes for which structural variations tend to indicate disease. FIG. 3 shows sixty-three genes in which copy number variation tends to indicate disease and seventeen cancer genes for which translocations are often indicative of cancer. The 63 genes in the copy number table are selectively screened for copy number variation. The 17 genes in the translocation table are analyzed for translocations. The panels shown in FIGS. 2 and 3 are just a few non-limiting examples of the types of panels that can be constructed and types of assays performed. Those skilled in the art will recognize that targeted panels can be created for many purposes, including targeting specific types of mutations or genes associated with specific types of cancer. A panel can be assayed for one class of mutation, or it can be screened for multiple types of mutations.

In another embodiment, a select panel of genes may be sequenced. A targeted approach may be useful when the patient has a known cancer, and so the assay can focus on the genes relevant to that cancer. For example, if a biopsy specimen is taken from a tumor in the breast, it would be more economical and efficient to assay the tumor DNA for a select panel of known breast cancer markers. The targeted approach can be used on ctDNA as well, when there is a reason to believe a patient has a specific type of cancer but biopsy is not feasible due to the type of cancer or location.

Another potential reason to use the targeted approach is for a patient with a family history of a certain type of cancer or a patient who is at elevated risk of certain cancers due to population genetics. A targeted gene panel may be used for testing a patient with exposure to certain risk factors. For example, it may be useful to test a patient for certain biomarkers that are associated with an elevated risk of lung cancer if that patient is a smoker.

In certain embodiments, methods of the invention are directed to analyzing genes known to be associated with breast cancer, bladder cancer, bone cancer, brain cancer, cervical cancer, esophageal cancer, Hodgkin Disease, kidney cancer, leukemia, liver cancer, lung cancer, lymphoma, ovarian cancer, prostate cancer, thyroid cancer, any other cancer known to have a genetic basis, or any combination thereof. Gene panels could be designed for new cancer genes as they are discovered.

Nucleic acids can be sequenced redundantly for confidence at coverage of 10X, 100X, 250X, 1000X, or more.

After the nucleic acid is sequenced, the tumor and normal sequencing reads may then be compiled into a consensus sequence. The consensus sequence of the sequencing reads may be generated by forming a contig with the obtained sequencing reads or by aligning the sequencing reads to a reference. The tumor and normal consensus sequences may be formed by the same method or different method. In certain embodiments, methods of the invention involve assembling a contig of the tumor sequence and a contig of the normal sequence to generate a consensus sequence for the tumor nucleic acid and the normal nucleic acid. Once generated, the consensus sequences of the tumor and normal can be compared to each other. In additional embodiments, methods of the invention involve aligning the tumor sequence reads to a reference to generate a tumor consensus sequence, and aligning the normal sequence reads to the reference to generate a normal consensus sequence, and then comparing the tumor and normal consensus sequences. After the consensus sequences are formed, the normal consensus sequence and consensus sequence are compared to identify variations.

Different ways of assembling a contig and generating a consensus sequence are discussed below.

A contig, generally, refers to the relationship between or among a plurality of segments of nucleic acid sequences, e.g., reads. Where sequence reads overlap, a contig can be represented as a layered image of overlapping reads. A contig is not defined by, nor limited to, any particular visual arrangement nor any particular arrangement within, for example, a text file or a database. A contig generally includes sequence data from a number of reads organized to correspond to a portion of a sequenced nucleic acid. A contig can include assembly results-such as a set of reads or information about their positions relative to each other or to a reference-displayed or stored. A contig can be structured as a grid, in which rows are individual sequence reads and columns include the base of each read that is presumed to align to that site. A consensus sequence can be made by identifying the predominant base in each column of the assembly. A contig according to the invention can include the visual display of reads showing them overlap (or not, e.g., simply abutting) one another. A contig can include a set of coordinates associated with a plurality of reads and giving the position of the reads relative to each other. A contig can include data obtained by transforming the sequence data of reads. For example, a Burrows-Wheeler transformation can be performed on the reads, and a contig can include the transformed data without necessarily including the untransformed sequences of the reads. A Burrows-Wheeler transform of nucleotide sequence data is described in U.S. Pub. 2005/0032095, herein incorporated by reference in its entirety.

Reads can be assembled into contigs by any method known in the art. Algorithms for the de novo assembly of a plurality of sequence reads are known in the art. One algorithm for assembling sequence reads is known as overlap consensus assembly. Overlap consensus assembly uses the overlap between sequence reads to create a link between them. The reads are generally linked by regions that overlap enough that non-random overlap is assumed. Linking together reads in this way produces a contig or an overlap graph in which each node corresponds to a read and an edge represents an overlap between two reads. Assembly with overlap graphs is described, for example, in U.S. Pat. 6,714,874.

In some embodiments, de novo assembly proceeds according to so-called greedy algorithms. For assembly according to greedy algorithms, one of the reads of a group of reads is selected, and it is paired with another read with which it exhibits a substantial amount of overlap-generally it is paired with the read with which it exhibits the most overlap of all of the other reads. Those two reads are merged to form a new read sequence, which is then put back in the group of reads and the process is repeated. Assembly according to a greedy algorithm is described, for example, in Schatz, et al., Genome Res., 20:1165-1173 (2010) and U.S. Pub. 2011/0257889, each of which is hereby incorporated by reference in its entirety.

In other embodiments, assembly proceeds by pairwise alignment, for example, exhaustive or heuristic (e.g., not exhaustive) pairwise alignment. Alignment, generally, is discussed in more detail below. Exhaustive pairwise alignment, sometimes called a "brute force" approach, calculates an alignment score for every possible alignment between every possible pair of sequences among a set. Assembly by heuristic multiple sequence alignment ignores certain mathematically unlikely combinations and can be computationally faster. One heuristic method of assembly by multiple sequence alignment is the so-called "divide-and-conquer" heuristic, which is described, for example, in U.S. Pub. 2003/0224384. Another heuristic method of assembly by multiple sequence alignment is progressive alignment, as implemented by the program ClustalW (see, e.g., Thompson, et al., Nucl. Acids. Res., 22:4673-80 (1994)). Assembly by multiple sequence alignment in general is discussed in Lecompte, O., et al., Gene 270:17-30 (2001); Mullan, L. J., Brief Bioinform., 3:303-5 (2002); Nicholas, H. B. Jr., et al., Biotechniques 32:572-91 (2002); and Xiong, G., Essential Bioinformatics, 2006, Cambridge University Press, New York, NY.

Assembly by alignment can proceed by aligning reads to each other or by aligning reads to a reference. For example, by aligning each read, in turn, to a reference genome, all of the reads are positioned in relationship to each other to create the assembly.

One method of assembling reads into contigs involves making a de Bruijn graph. De Bruijn graphs reduce the computation effort by breaking reads into smaller sequences of DNA, called k-mers, where the parameter k denotes the length in bases of these sequences. In a de Bruijn graph, all reads are broken into k-mers (all subsequences of length k within the reads) and a path between the k-mers is calculated. In assembly according to this method, the reads are represented as a path through the k-mers. The de Bruijn graph captures overlaps of length k-1 between these k-mers and not between the actual reads. Thus, for example, the sequencing CATGGA could be represented as a path through the following 2-mers: CA, AT, TG, GG, and GA. The de Bruijn graph approach handles redundancy well and makes the computation of complex paths tractable. By reducing the entire data set down to k-mer overlaps, the de Bruijn graph reduces the high redundancy in short-read data sets. The maximum efficient k-mer size for a particular assembly is determined by the read length as well as the error rate. The value of the parameter k has significant influence on the quality of the assembly. Estimates of good values can be made before the assembly, or the optimal value can be found by testing a small range of values. Assembly of reads using de Bruijn graphs is described in U.S. Pub. 2011/0004413, U.S. Pub. 2011/0015863, and U.S. Pub. 2010/0063742, each of which are herein incorporated by reference in their entirety.

Other methods of assembling reads into contigs according to the invention are possible. For example, the reads may contain barcode information inserted into template nucleic acid during sequencing. In certain embodiments, reads are assembled into contigs by reference to the barcode information. For example, the barcodes can be identified and the reads can be assembled by positioning the barcodes together.

Assembly of reads into contigs is further discussed in Husemann, P. and Stoye, J, Phylogenetic Comparative Assembly, 2009, Algorithms in Bioinformatics: 9th International Workshop, pp. 145-156, Salzberg, S., and Warnow, T., Eds. Springer-Verlag, Berlin Heidelberg. Some exemplary methods for assembling reads into contigs are described, for example, in U.S. Pat. 6,223,128, U.S. Pub. 2009/0298064, U.S. Pub. 2010/0069263, and U.S. Pub. 2011/0257889, each of which is incorporated by reference herein in its entirety.

Computer programs for assembling reads are known in the art. Such assembly programs can run on a single general-purpose computer, on a cluster or network of computers, or on a specialized computing devices dedicated to sequence analysis.

Assembly can be implemented, for example, by the program 'The Short Sequence Assembly by k-mer search and 3' read Extension ' (SSAKE), from Canada's Michael Smith Genome Sciences Centre (Vancouver, B.C., CA) (see, e.g., Warren, R., et al., Bioinformatics, 23:500-501 (2007)). SSAKE cycles through a table of reads and searches a prefix tree for the longest possible overlap between any two sequences. SSAKE clusters reads into contigs.

Another read assembly program is Forge Genome Assembler, written by Darren Platt and Dirk Evers and available through the SourceForge web site maintained by Geeknet (Fairfax, VA) (see, e.g., DiGuistini, S., et al., Genome Biology, 10:R94 (2009)). Forge distributes its computational and memory consumption to multiple nodes, if available, and has therefore the potential to assemble large sets of reads. Forge was written in C++ using the parallel MPI library. Forge can handle mixtures of reads, e.g., Sanger, 454, and Illumina reads.

Assembly through multiple sequence alignment can be performed, for example, by the program Clustal Omega, (Sievers F., et al., Mol Syst Biol 7 (2011)), ClustalW, or ClustalX (Larkin M.A., et al., Bioinformatics, 23, 2947-2948 (2007)) available from University College Dublin (Dublin, Ireland).

Another exemplary read assembly program known in the art is Velvet, available through the web site of the European Bioinformatics Institute (Hinxton, UK) (Zerbino D.R. et al., Genome Research 18(5):821-829 (2008)). Velvet implements an approach based on de Bruijn graphs, uses information from read pairs, and implements various error correction steps.

Read assembly can be performed with the programs from the package SOAP, available through the website of Beijing Genomics Institute (Beijing, CN) or BGI Americas Corporation (Cambridge, MA). For example, the SOAPdenovo program implements a de Bruijn graph approach. SOAP3/GPU aligns short reads to a reference sequence.

Another read assembly program is ABySS, from Canada's Michael Smith Genome Sciences Centre (Vancouver, B.C., CA) (Simpson, J.T., et al., Genome Res., 19(6):1117-23 (2009)). ABySS uses the de Bruijn graph approach and runs in a parallel environment.

Read assembly can also be done by Roche's GS De Novo Assembler, known as gsAssembler or Newbler (NEW assemBLER), which is designed to assemble reads from the Roche 454 sequencer (described, e.g., in Kumar, S. et al., Genomics 11:571 (2010) and Margulies, et al., Nature 437:376-380 (2005)). Newbler accepts 454 Flx Standard reads and 454 Titanium reads as well as single and paired-end reads and optionally Sanger reads. Newbler is run on Linux, in either 32 bit or 64 bit versions. Newbler can be accessed via a command-line or a Java-based GUI interface.

Cortex, created by Mario Caccamo and Zamin Iqbal at the University of Oxford, is a software framework for genome analysis, including read assembly. Cortex includes cortex_con for consensus genome assembly, used as described in Spanu, P.D., et al., Science 330(6010):1543-46 (2010). Cortex includes cortex_var for variation and population assembly, described in Iqbal, et al., De novo assembly and genotyping of variants using colored de Bruijn graphs, Nature Genetics (in press), and used as described in Mills, R.E., et al., Nature 470:59-65 (2010). Cortex is available through the creators' web site and from the SourceForge web site maintained by Geeknet (Fairfax, VA).

Other read assembly programs include RTG Investigator from Real Time Genomics, Inc. (San Francisco, CA); iAssembler (Zheng, et al., BMC Bioinformatics 12:453 (2011)); TgiCL Assembler (Pertea, et al., Bioinformatics 19(5):651-52 (2003)); Maq (Mapping and Assembly with Qualities) by Heng Li, available for download through the SourceForge website maintained by Geeknet (Fairfax, VA); MIRA3 (Mimicking Intelligent Read Assembly), described in Chevreux, B., et al., Genome Sequence Assembly Using Trace Signals and Additional Sequence Information, 1999, Computer Science and Biology: Proceedings of the German Conference on Bioinformatics (GCB) 99:45-56; PGA4genomics (described in Zhao F., et al., Genomics. 94(4):284-6 (2009)); and Phrap (described, e.g., in de la Bastide, M. and McCombie, W. R., Current Protocols in Bioinformatics, 17:11.4.1-11.4.15 (2007)). CLC cell is a de Bruijn graph-based computer program for read mapping and de novo assembly of NGS reads available from CLC bio Germany (Muehltal, Germany).

Assembly of reads produces one or more contigs. In the case of a homozygous or single target sequencing, a single contig will be produced. In the case of a heterozygous diploid target, a rare somatic mutation, or a mixed sample, for example, two or more contigs can be produced. Each contig includes information from the reads that make up that contig.

Assembling the reads into contigs is conducive to producing a consensus sequence corresponding to each contig. In certain embodiments, a consensus sequence refers to the most common, or predominant, nucleotide at each position from among the assembled reads. A consensus sequence can represent an interpretation of the sequence of the nucleic acid represented by that contig.

Alignment, as used herein, generally involves placing one sequence along another sequence, iteratively introducing gaps along each sequence, scoring how well the two sequences match, and preferably repeating for various positions along the reference. The best-scoring match is deemed to be the alignment and represents an inference about the historical relationship between the sequences. In an alignment, a base in the read alongside a non-matching base in the reference indicates that a substitution mutation has occurred at that point. Similarly, where one sequence includes a gap alongside a base in the other sequence, an insertion or deletion mutation (an "indel") is inferred to have occurred. When it is desired to specify that one sequence is being aligned to one other, the alignment is sometimes called a pairwise alignment. Multiple sequence alignment generally refers to the alignment of two or more sequences, including, for example, by a series of pairwise alignments.

In some embodiments, scoring an alignment involves setting values for the probabilities of substitutions and indels. When individual bases are aligned, a match or mismatch contributes to the alignment score by a substitution probability, which could be, for example, 1 for a match and 0.33 for a mismatch. An indel deducts from an alignment score by a gap penalty, which could be, for example, -1. Gap penalties and substitution probabilities can be based on empirical knowledge or a priori assumptions about how sequences mutate. Their values affect the resulting alignment. Particularly, the relationship between the gap penalties and substitution probabilities influences whether substitutions or indels will be favored in the resulting alignment.

Stated formally, an alignment represents an inferred relationship between two sequences, x and y. For example, in some embodiments, an alignment A of sequences x and y maps x and y respectively to another two strings x' and y' that may contain spaces such that: (i) |x'l=|y'|; (ii) removing spaces from x' and y' should get back x and y, respectively; and (iii) for any i, x'[i ] and y'[i ] cannot be both spaces.

A gap is a maximal substring of contiguous spaces in either x' or y'. An alignment A can include the following three kinds of regions: (i) matched pair (e.g., x'[i]=y'[i]; (ii) mismatched pair, (e.g., x'[i]≠y'[i] and both are not spaces); or (iii) gap (e.g., either x'[i..j] or y'[i..j] is a gap). In certain embodiments, only a matched pair has a high positive score a. In some embodiments, a mismatched pair generally has a negative score b and a gap of length r also has a negative score g+rs where g, s<0. For DNA, one common scoring scheme (e.g. used by BLAST) makes score a=1, score b=-3, g=-5 and s=-2. The score of the alignment A is the sum of the scores for all matched pairs, mismatched pairs and gaps. The alignment score of x and y can be defined as the maximum score among all possible alignments of x and y.

In some embodiments, any pair has a score a defined by a 4x4 matrix B of substitution probabilities. For example, B(i,i)=1 and 0< B(i,j)i<>j <1 is one possible scoring system. For instance, where a transition is thought to be more biologically probable than a transversion, matrix B could include B(C,T)=.7 and B(A,T)=.3, or any other set of values desired or determined by methods known in the art.

Alignment according to some embodiments of the invention includes pairwise alignment. A pairwise alignment, generally, involves-for sequence Q (query) having m characters and a reference genome T (target) of n characters-finding and evaluating possible local alignments between Q and T. For any 1≤i≤n and 1≤j≤m, the largest possible alignment score of T[h..i] and Q[k..j], where h≤i and k≤j, is computed (i.e. the best alignment score of any substring of T ending at position i and any substring of Q ending at position j ). This can include examining all substrings with cm characters, where c is a constant depending on a similarity model, and aligning each substring separately with Q. Each alignment is scored, and the alignment with the preferred score is accepted as the alignment. In some embodiments an exhaustive pairwise alignment is performed, which generally includes a pairwise alignment as described above, in which all possible local alignments (optionally subject to some limiting criteria) between Q and T are scored.

In some embodiments, pairwise alignment proceeds according to dot-matrix methods, dynamic programming methods, or word methods. Dynamic programming methods generally implement the Smith-Waterman (SW) algorithm or the Needleman-Wunsch (NW) algorithm. Alignment according to the NW algorithm generally scores aligned characters according to a similarity matrix S(a,b) (e.g., such as the aforementioned matrix B) with a linear gap penalty d. Matrix S(a,b) generally supplies substitution probabilities. The SW algorithm is similar to the NW algorithm, but any negative scoring matrix cells are set to zero. The SW and NW algorithms, and implementations thereof, are described in more detail in U.S. Pat. 5,701,256 and U.S. Pub. 2009/0119313, both herein incorporated by reference in their entirety. Computer programs known in the art for implementing these methods are described in more detail below.

An alignment according to the invention can be performed using any suitable computer program known in the art.

One exemplary alignment program, which implements a BWT approach, is Burrows-Wheeler Aligner (BWA) available from the SourceForge web site maintained by Geeknet (Fairfax, VA). BWA can align reads, contigs, or consensus sequences to a reference. BWT occupies 2 bits of memory per nucleotide, making it possible to index nucleotide sequences as long as 4G base pairs with a typical desktop or laptop computer. The pre-processing includes the construction of BWT (i.e., indexing the reference) and the supporting auxiliary data structures.

BWA implements two different algorithms, both based on BWT. Alignment by BWA can proceed using the algorithm bwa-short, designed for short queries up to ~200bp with low error rate (<3%) (Li H. and Durbin R. Bioinformatics, 25:1754-60 (2009)). The second algorithm, BWA-SW, is designed for long reads with more errors (Li H. and Durbin R. (2010) Fast and accurate long-read alignment with Burrows-Wheeler Transform. Bioinformatics, Epub.). The BWA-SW component performs heuristic Smith-Waterman-like alignment to find high-scoring local hits. One skilled in the art will recognize that bwa-sw is sometimes referred to as "bwa-long", "bwa long algorithm", or similar. Such usage generally refers to BWA-SW.

An alignment program that implements a version of the Smith-Waterman algorithm is MUMmer, available from the SourceForge web site maintained by Geeknet (Fairfax, VA). MUMmer is a system for rapidly aligning entire genomes, whether in complete or draft form (Kurtz, S., et al., Genome Biology, 5:R12 (2004); Delcher, A.L., et al., Nucl. Acids Res., 27:11 (1999)). For example, MUMmer 3.0 can find all 20-basepair or longer exact matches between a pair of 5-megabase genomes in 13.7 seconds, using 78 MB of memory, on a 2.4 GHz Linux desktop computer. MUMmer can also align incomplete genomes; it can easily handle the 100s or 1000s of contigs from a shotgun sequencing project, and will align them to another set of contigs or a genome using the NUCmer program included with the system. If the species are too divergent for a DNA sequence alignment to detect similarity, then the PROmer program can generate alignments based upon the six-frame translations of both input sequences.

Another exemplary alignment program according to embodiments of the invention is BLAT from Kent Informatics (Santa Cruz, CA) (Kent, W.J., Genome Research 4: 656-664 (2002)). BLAT (which is not BLAST) keeps an index of the reference genome in memory such as RAM. The index includes of all non-overlapping k-mers (except optionally for those heavily involved in repeats), where k=11 by default. The genome itself is not kept in memory. The index is used to find areas of probable homology, which are then loaded into memory for a detailed alignment.

Another alignment program is SOAP2, from Beijing Genomics Institute (Beijing, CN) or BGI Americas Corporation (Cambridge, MA). SOAP2 implements a 2-way BWT (Li et al., Bioinformatics 25(15):1966-67 (2009); Li, et al., Bioinformatics 24(5):713-14 (2008)).

Another program for aligning sequences is Bowtie (Langmead, et al., Genome Biology, 10:R25 (2009)). Bowtie indexes reference genomes by making a BWT.

Other exemplary alignment programs include: Efficient Large-Scale Alignment of Nucleotide Databases (ELAND) or the ELANDv2 component of the Consensus Assessment of Sequence and Variation (CASAVA) software (Illumina, San Diego, CA); RTG Investigator from Real Time Genomics, Inc. (San Francisco, CA); Novoalign from Novocraft (Selangor, Malaysia); Exonerate, European Bioinformatics Institute (Hinxton, UK) (Slater, G., and Birney, E., BMC Bioinformatics 6:31 (2005)), Clustal Omega, from University College Dublin (Dublin, Ireland) (Sievers F., et al., Mol Syst Biol 7, article 539 (2011)); ClustalW or ClustalX from University College Dublin (Dublin, Ireland) (Larkin M.A., et al., Bioinformatics, 23, 2947-2948 (2007)); and FASTA, European Bioinformatics Institute (Hinxton, UK) (Pearson W.R., et al., PNAS 85(8):2444-8 (1988); Lipman, D.J., Science 227(4693):1435-41 (1985)).

After the comparison of the normal and tumor consensus sequences, the tumor sequence is filtered based on the comparison. The filtering is based on differences between the sequences, where loci that do not meet a certain threshold (i.e., the sequences are the same or similar) are excluded from further analysis. The purpose of excluding these similar sequences is to remove sequences from the subsequent analysis that are normally associated with that particular patient's genome, or that are not sufficiently different than the patient's normal genome. This step therefore removes the false-positives (i.e. mutation calls that are not specific to the tumor) from the assay by focusing only on non-normal variations.

In certain embodiments, a threshold is used to determine whether a variation between a portion of the tumor sequence and a corresponding portion of the normal sequence is significant enough to be classified as a variant specific to the tumor. Due to the many types of sequence variations that are possible when comparing the tumor sequence and normal sequence, and the different effects those variations have on gene expression, different thresholds apply. In certain embodiments, any variation in the tumor sequence as compared to the normal sequence is identified as a variant specific to the tumor, and may be classified as a tumor specific biomarker. In other embodiments, variant sequences specific to the tumor are identified based on their similarity or dissimilarity to the normal sequence. For example, a portion of the tumor sequence may be classified as a variant specific to the tumor because it varies from a corresponding segment of the normal sequence to a degree of 75%, 80%, 85%, 90%, 95%, 96%, 97%, 98%, 99%, 99.1%, 99.5%, 99.6%, 99.7%, 99.8%, 99.9%, etc. In another example, a portion of the tumor sequence may be classified as normal because it is similar to a corresponding segment of the normal sequence to a degree of 75%, 80%, 85%, 90%, 95%, 96%, 97%, 98%, 99%, 99.1%, 99.5%, 99.6%, 99.7%, 99.8%, 99.9%, etc.

Depending on the threshold chosen, the filtered tumor sequence (i.e. tumor-specific variant) may require additional analysis to identify mutations within the filtered sequence. For example, a threshold may be chosen such that only exact matches of a certain nucleotide length between the normal and tumor are filtered out from subsequent analysis. While this eliminates normal matches of a certain kind, some portions of the filtered sequence may not be indicative of a tumor mutation by virtue of the threshold chosen. In such example, the filtered sequence may be compared to a tumor reference in order to confirm locations of tumor-specific mutations within the filtered sequence.

In other example, non-quantitative thresholds may be used to classify a portion of the tumor sequence as a variant specific to the tumor, such as whether a mutation results in a change in the resultant protein sequence.

In certain embodiments, the threshold chosen is the same or different for different types of mutations. For example, the threshold for single nucleotide polymorphisms may be different from the threshold chosen for translocations.

Some mutations, such as copy number variation, for example, have a quantitative threshold. In an embodiment, copy numbers that fall within a threshold of 20% above or below normal are removed from analysis. Copy number variation within this range is not considered to be statistically significant.

The threshold for single-nucleotide polymorphisms (SNPs) is qualitative. These mutations are filtered out if they do not change the protein sequence, as they are not considered significant to gene expression. SNPs that result in an incorrect protein or an early stop codon are always called mutations for the purpose of the invention.

Other mutations that have a clearly deleterious effect on gene expression are automatically called mutations. For example, insertions into the coding sequence and deletions from the coding sequence are automatic calls. Insertions and deletions in non-coding regions are filtered out if they are fewer than 10 nucleotides. Translocations, on the other hand, are automatically called mutations because of their significant relationship with cancer.

Once the tumor sequence is filtered based on the various thresholds described above, it can be compared to a reference sequence to identify a mutation. The reference sequence may be a normal reference, such as a representative sequence assembled from sequencing and compiling nucleic acid from a number of healthy donors. The reference sequence can also be a disease sequence, such as a sequence assembled from sequencing and compiling nucleic acid from donors having a disease, such as cancer. If a patient's nucleic acid sample has been sequenced for a panel of prostate cancer genes, for example, the filtered result can be compared to a prostate cancer reference sequence to identify which mutations are known.

Various cancer reference sequences are available and known to those of skill in the art. By comparing the filtered sequence to a tumor reference, the mutations specific to the patient can be identified, while reducing the false positives that would have remained in the set without the filtering. Methods of the invention include the use of germline databases including the Exome Sequencing Project (ESP) as well as other ongoing large scale germline analyses such as the Genomics England 100,000 genomes project and the Human Longevity sequencing initiative. Tools such as CHASM (Cancer-specific High-throughput Annotation of Somatic Mutations), SIFT, PolyPhen, and others could be used to predict whether a somatic mutation is likely a driver or passenger even in the absence of normal DNA.

After assessing the filtered tumor sequence reads, a proper diagnosis and treatment regimen can be developed that is patient-specific. Methods of the invention are useful for identifying known genes with potential clinical significance, and assessing clinical actionability. Some well-known mutations that are identified can be readily classified as cancerous mutations. However, the individualized filtered results of the invention allow for characterizing the other identified sequence variations in the patient's genetic sequence as causative or representative of the cancer. This allows for more accurate diagnosis of the patient's cancer. A treatment regimen can be designed that is tailored specifically to the mutations identified in the filtered sequence. The invention prevents misdiagnosis based on, for example, a false-positive mutation call at a locus where the locus actually represents a normal sequence variation in the patient's genome.

Clinical actionability can be assessed in a number of ways. For example, genes can be identified that are associated with FDA-approved therapies (http://www.fda.gov/Drugs/), or a literature search can be conducted to identify published prospective and retrospective clinical studies pertaining to genomic alterations of each gene and their association with outcome for cancer patients. Genes that served as targets for specific agents or were predictors of response or resistance to cancer therapies when mutated may be considered actionable. Alternatively, clinical trials can be identified (http://clinicaltrials.gov/) that specify altered genes within the inclusion criteria. In all cases, the tumor type relevant to the FDA approval or studied in the clinical trials was determined to allow the clinical information to be matched to the mutational data by both gene and cancer type.

The invention is also useful in the continuing care of a cancer patient. After beginning a treatment regimen, the patient's tumor sequence can be analyzed again using the same methods. This second analysis can determine whether there are more or fewer mutations, which is indicative of whether the cancer is progressing.

A technique for quality control that can be used with the invention is comparing the next generation sequencing data to a Sanger sequencing reference. Sanger reference data is known to have greater accuracy than next-generation sequencing data, and thus can be used to confirm the legitimacy of variations. The NGS sequencing reads of a patient's tumor sample, a patient's normal sample, or both may be filtered against a Sanger reference prior to being compared to each other to identify tumor-specific mutations. In some embodiments, sections of the NGS sequencing reads of a patient's tumor sample which have been determined to contain a tumor specific mutation through comparison to NGS sequencing reads of a patient's normal sample may subsequently be filtered against a Sanger sequencing reference in order to validate the mutation. Methods and systems of comparing next generation sequence reads with a Sanger sequencing reference are described in the co-pending application entitled "Increasing Sensitivity and Specificity in Next-Generation Sequencing Based Patient-Specific Genomic Analysis," filed on concurrently herewith.

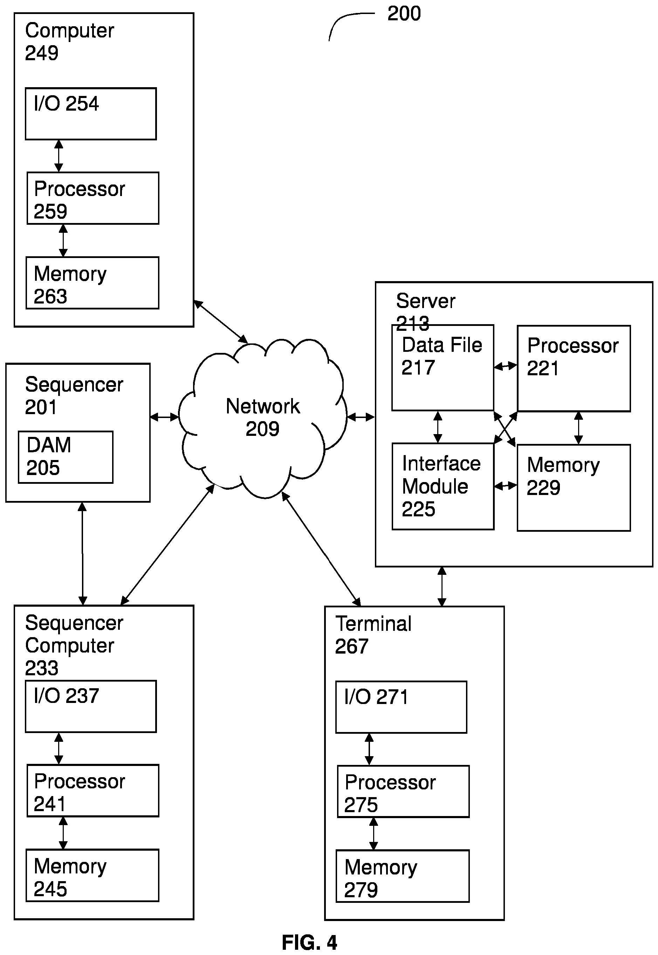

FIG. 4 diagrams a system 200 of the invention. As one skilled in the art would recognize as necessary or best-suited for performance of the methods of the invention and sequence assembly in general, computer system 200 or machines of the invention include one or more processors (e.g., a central processing unit (CPU) a graphics processing unit (GPU) or both), a main memory and a static memory, which communicate with each other via a bus.

In an exemplary embodiment shown in FIG. 4, system 200 can include a sequencer 201 with data acquisition module 205 to obtain sequence read data. Sequencer 201 may optionally include or be operably coupled to its own, e.g., dedicated, sequencer computer 233 (including an input/output mechanism 237, one or more of processor 241 and memory 245). Additionally or alternatively, sequencer 201 may be operably coupled to a server 213 or computer 249 (e.g., laptop, desktop, or tablet) via network 209. Computer 249 includes one or more processor 259 and memory 263 as well as an input/output mechanism 254. Where methods of the invention employ a client/server architecture, steps of methods of the invention may be performed using server 213, which includes one or more of processor 221 and memory 229, capable of obtaining data, instructions, etc., or providing results via interface module 225 or providing results as a file 217. Server 213 may be engaged over network 209 through computer 249 or terminal 267, or server 213 may be directly connected to terminal 267, including one or more processor 275 and memory 279, as well as input/output mechanism 271.

System 200 or machines according to the invention may further include, for any of I/O 249, 237, or 271 a video display unit (e.g., a liquid crystal display (LCD) or a cathode ray tube (CRT)). Computer systems or machines according to the invention can also include an alphanumeric input device (e.g., a keyboard), a cursor control device (e.g., a mouse), a disk drive unit, a signal generation device (e.g., a speaker), a touchscreen, an accelerometer, a microphone, a cellular radio frequency antenna, and a network interface device, which can be, for example, a network interface card (NIC), Wi-Fi card, or cellular modem.

Memory 263, 245, 279, or 229 according to the invention can include a machine-readable medium on which is stored one or more sets of instructions (e.g., software) embodying any one or more of the methodologies or functions described herein. The software may also reside, completely or at least partially, within the main memory and/or within the processor during execution thereof by the computer system, the main memory and the processor also constituting machine-readable media.

The software may further be transmitted or received over a network via the network interface device.

While the machine-readable medium can in an exemplary embodiment be a single medium, the term "machine-readable medium" should be taken to include a single medium or multiple media (e.g., a centralized or distributed database, and/or associated caches and servers) that store the one or more sets of instructions. The term "machine-readable medium" shall also be taken to include any medium that is capable of storing, encoding or carrying a set of instructions for execution by the machine and that cause the machine to perform any one or more of the methodologies of the present invention. The term "machine-readable medium" shall accordingly be taken to include, but not be limited to, solid-state memories (e.g., subscriber identity module (SIM) card, secure digital card (SD card), micro SD card, or solid-state drive (SSD)), optical and magnetic media, and any other tangible storage media.

### Example

To evaluate the clinical utility of large-scale cancer genome analyses as disclosed herein, whole exome and targeted next generation sequencing analyses were performed in tumor and normal samples from cancer patients. Matched tumor and normal data were analyzed together as well as separately for somatic mutation detection, potential clinical actionability, and identification of predisposing alterations.

Eight-hundred fifteen (815) tumor-normal paired samples from patients of fifteen tumor types were comprehensively evaluated. Genomic alterations were identified using next generation sequencing approaches of whole exomes or 111 targeted genes that were validated with sensitivities of >95% and >99%, respectively, and a specificity of >99.9%. Those analyses revealed an average of 140 and 4.3 somatic mutations per exome and targeted analyses, respectively. Over 75% of cases had somatic alterations in genes associated with known therapies or current clinical trials, with the majority of actionable genes infrequently altered in any tumor type. Analyses of matched normal DNA identified germline alterations in cancer predisposing genes in 3% of patients with apparently sporadic cancers.

To systematically assess somatic alterations in tumor samples, capture probes were designed for a set of 111 clinically relevant genes known in the art. Those genes were: ABL1; AKT1; AKT2; ALK; APC; AR; ARID1A; ARID1B; ASXL1; ATM; ATRX; BAP1; BRAF; BRCA1; BRCA2; CBL; CCND1; CCNE1; CDH1; CDK4; CDK6; CDKN2A; CEBPA; CREBBP; CTNNB1; DAXX; DNMT3A; EGFR; ERBB2; ERBB3; ERBB4; EZH2; FBXW7; FGFR2; FGFR3; FGFR4; FLT3; FOXL2; GATA1; GATA2; GNA11; GNAQ; GNAS; HNF1A; HRAS; IDH1; IDH2; IGF1R; IGF2R; IKZF1; JAK1; JAK2; JAK3; KDR; KIT; KRAS; MAML1; MDM2; MDM4; MED12; MEN1; MET; MLH1; MLL; MPL; MSH2; MSH6; MYC; MYCN; MYD88; NF1; NF2; NOTCH1; NOTCH2; NOTCH3; NOTCH4; NPM1; NRAS; PALB2; PAX5; PBRM1; PDGFRA; PDGFRB; PIK3CA; PIK3R1; PMS2; PTCH1; PTEN; PTPN11; RB1; RET; RNF43; ROS1; RUNX1; SF3B1; SMAD2; SMAD3; SMAD4; SMARCB1; SMO; STAG2; STK11; TET2; TGFBR2; TNFAIP3; TP53; TSC1; TSC2; TSHR; VHL; and WT1.

Those regions or the complete set of coding genes (20,766 genes) were sequenced using next generation sequencing approaches. Those data were aligned to the human reference sequence and annotated using the Consensus Coding DNA Sequences (CCDS), RefSeq and Ensembl databases.

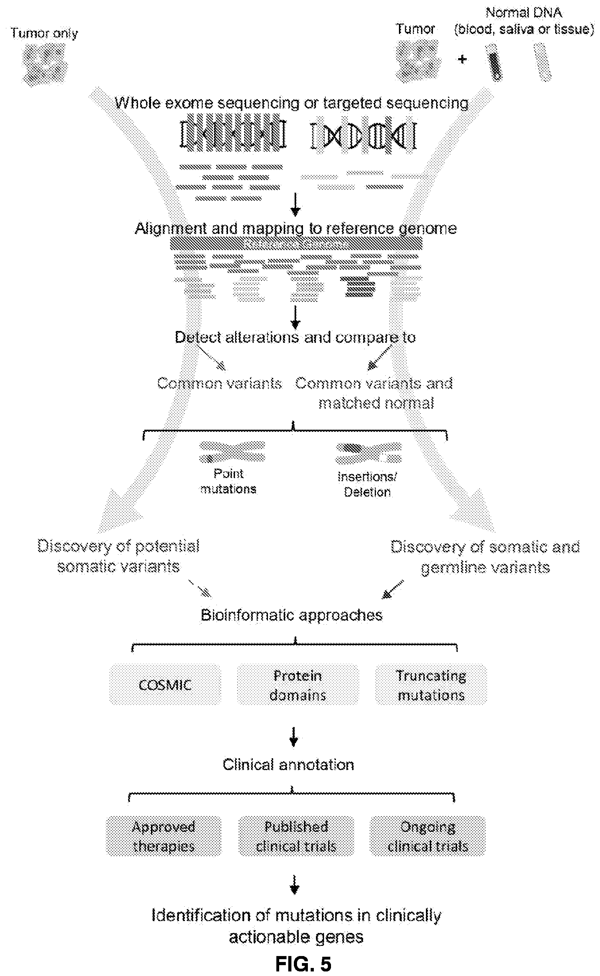

FIG. 5 diagrams whole exome or targeted next generation sequencing analyses. The left side of the diagram shows tumor-only approach, and the right side of the diagram shows a matched tumor-normal approach for identifying sequence alterations. Bioinformatic methods to separate germline and somatic changes include comparison to dbSNP, COSMIC, and kinase domain databases. Identified gene alterations can be compared to databases of established and experimental therapies to identify potential clinical actionability and predisposing alterations. Those methods are discussed in greater detail below.

Tumor and normal data were compared to identify somatic and germline alterations using the VariantDx software pipeline, focusing on single base substitutions as well as small insertions and deletions. VariantDx examines sequence alignments of tumor samples against a matched normal while applying filters to exclude alignment and sequencing artifacts. In brief, an alignment filter was applied to exclude quality failed reads, unpaired reads, and poorly mapped reads in the tumor. A base quality filter was applied to limit inclusion of bases with reported phred quality score > 30 for the tumor and > 20 for the normal. A mutation in the tumor was identified as a candidate somatic mutation only when: (i) distinct paired reads contained the mutation in the tumor; (ii) the number of distinct paired reads containing a particular mutation in the tumor was at least 2% of the total distinct read pairs for targeted analyses and 10% of read pairs for exome; (iii) the mismatched base was not present in >1% of the reads in the matched normal sample as well as not present in a custom database of common germline variants derived from dbSNP; and (iv) the position was covered in both the tumor and normal. Mutations arising from misplaced genome alignments, including paralogous sequences, were identified and excluded by searching the reference genome.

Candidate somatic mutations were further filtered based on gene annotation to identify those occurring in protein coding regions. Functional consequences were predicted using snpEff and a custom database of CCDS, RefSeq and Ensembl annotations using the latest transcript versions available on hg18 from UCSC (https://genome.ucsc.edu/). Predictions were ordered to prefer transcripts with canonical start and stop codons and CCDS or Refseq transcripts over Ensembl when available.

Stringent criteria were used to ensure sufficient coverage at analyzed bases and to exclude mapping and sequencing errors. All candidate somatic alterations were visually inspected to remove remaining artifactual changes.

Analysis of samples using both whole-exome Sanger sequencing and next generation sequencing was used to demonstrate that the next generation sequencing and bioinformatic approaches were able to detect somatic mutations in frozen and formalin fixed paraffin embedded tumor (FFPE) tissues with high sensitivity and specificity and to accurately distinguish between somatic and germline alterations.

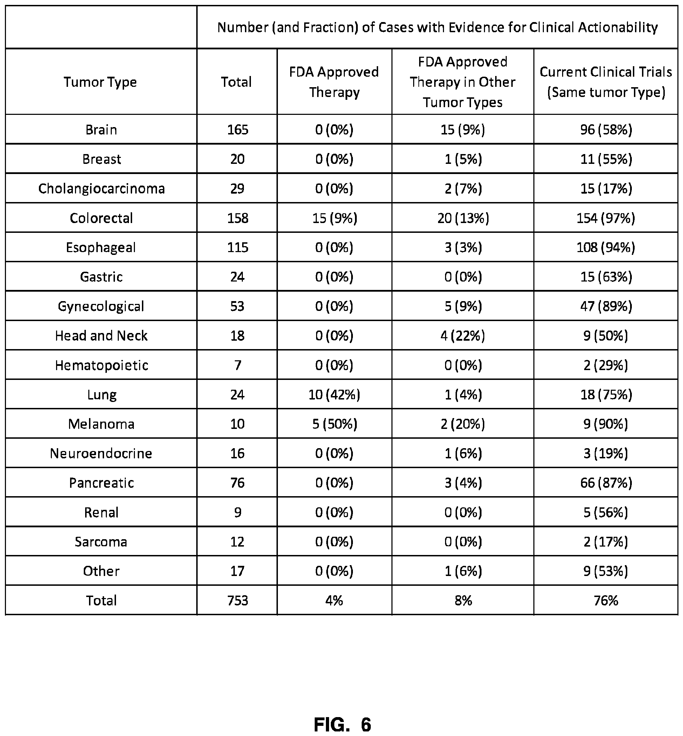

Using the above approach, matched tumor and normal specimens were analyzed from 815 patients with a variety of tumor types. A total of 105,672 somatic alterations were identified, with an average of 4.34 somatic mutations (range 0 to 29) in the targeted analyses and an average of 140 somatic alterations (range 1 to 6219) in the exome analyses. The number of somatic alterations in various tumor types was largely consistent with previous analyses of cancer exomes. To explore whether genetic alterations may be useful clinically, mutant genes were observed in individual cases to assess whether they would be clinically actionable using existing or investigational therapies. Altered genes were examined that were associated with: 1) FDA-approved therapies for oncologic indications; 2) therapies in published prospective clinical studies; and 3) ongoing clinical trials for patients with tumor types analyzed. Through these analyses somatic alterations were identified in genes with potentially actionable consequences in 580 of the 753 patients analyzed (77%) (as shown in FIG. 6). Those genes with known tumor types and therapies include: TP53; KRAS; PIK3CA; IDH1; EGFR; NF1; BRAF; BRCA2; ROS1; FLT4; PTEN; ALK; TSC2; FANCM; PTCH1; BRCA1; ERBB2; MET; NRAS; TSC1; PMS2; RET; NTRK1; KIT; FANCI; MSH6; SMO; FGFR3; MSH2; CTNNB1; FANCG; FLT3; JAK2; VHL; FANCC; MLH1; FANCA; FANCD2; AKT1; FANCB; FANCL; FANCF; CDKN2A; HRAS; GNA11; MAP2K1; and PDGFRA.

Some tumor types such as colorectal and melanoma had a much higher fraction of actionable changes than others. More than 90% of genes with potentially actionable alterations were mutated in <5% of individual tumors, suggesting that actionable changes are predominantly different among cancer patients.

FIG. 6 shows a number and fraction of cases with evidence for clinical actionability by tissue type. Although the fraction of patients that had at least one actionable alteration was high, most of the actionable changes were associated with current clinical trials (67%) rather than established or investigative therapies (33%).

To determine whether the disclosed analyses identified cancer predisposing changes in the genomes of apparently sporadic cancer patients, a set of 84 genes associated with known cancer predisposition syndromes was assessed in DNA from blood, saliva, or other normal tissue of the 815 cancer patients. Those genes were: ALK; APC; ATM; AXIN2; BAP1; BLM; BMPR1A; BRCA1; BRCA2; BRIP1; BUB1B; CDC73; CDH1; CDK4; CDKN2A; CHEK2; CREBBP; CYLD; DDB2; DICER1; EP300; ERCC2; ERCC3; ERCC4; ERCC5; EXT1; EXT2; FANCA; FANCB; FANCC; FANCD2; FANCE; FANCF; FANCG; FANCI; FANCL; FANCM; FH; FLCN; GPC3; KIT; MEN1; MET; MLH1; MSH2; MSH6; MUTYH; NBN; NF1; NF2; PALB2; PDGFRA; PHOX2B; PMS2; POLD1; POLE; POLH; POT1; PRKAR1A; PRSS1; PTCH1; PTEN; RAD51C; RB1; RECQL4; RET; SBDS; SDHAF2; SDHB; SDHC; SDHD; SMAD4; STK11; SUFU; TERT; TP53; TSC1; TSC2; VHL; WAS; WRN; WT1; XPA; and XPC.

To conservatively identify protein-altering changes in those genes the analysis focused on truncating alterations, including insertions or deletions resulting in a frameshift, splice site changes, and nonsense alterations. Through those analyses, 27 of the 815 patients (~3%) were identified with truncating alterations in those genes. All but one of those cases was not previously known to have a cancer predisposing alteration in its germline. Fifteen mutations were predicted to be pathogenic or likely pathogenic based on previous publications. Examples of germline alterations included changes in genes in expected tumor types, such as BRCA1 alterations in breast and ovarian cancer patients and a nonsense mutation (50Q>X) in CDKN2A in a melanoma case. However, less well described examples were also detected, including BRCA2 alterations in other solid tumor types such colorectal and cholangiocarcinoma, ATM changes in esophageal cancer, FANC alterations in a variety of tumor types, and alterations in the BRIP1 (BRCA1 interacting protein C-terminal helicase 1) gene in a cholangiocarcinoma (800Y>X) and in an anal cancer case (624S>X).

Using methods of the present invention, other bioinformatics approaches that do not use a matched normal for separating somatic from germline mutations can be evaluated. In contrast to the results for the tumor-normal matched approach described above, a tumor-only sequencing approach followed by bioinformatic removal of common germline variants is less accurate and precise, but still valuable. Furthermore, in some instances, tumor-only sequencing with post sequencing clean-up will be the best available option because of a lack of a matching normal sample).

As discussed below, a tumor-only analysis of the same tumor sample leads to a 31% and 65% false discovery rate in alterations identified in targeted and exome analyses, respectively, including potentially actionable genes. Those data suggest that matched tumor-normal sequencing analyses are essential for precise identification and interpretation of somatic and germline alterations and have important implications for the diagnostic and therapeutic management of cancer patients. Furthermore, because of the superior performance (high specificity and sensitivity) of the described tumor-matched-normal methods described herein, the tumor-matched-normal methods may also be used as a quality-control check against other methods of evaluating tumors.

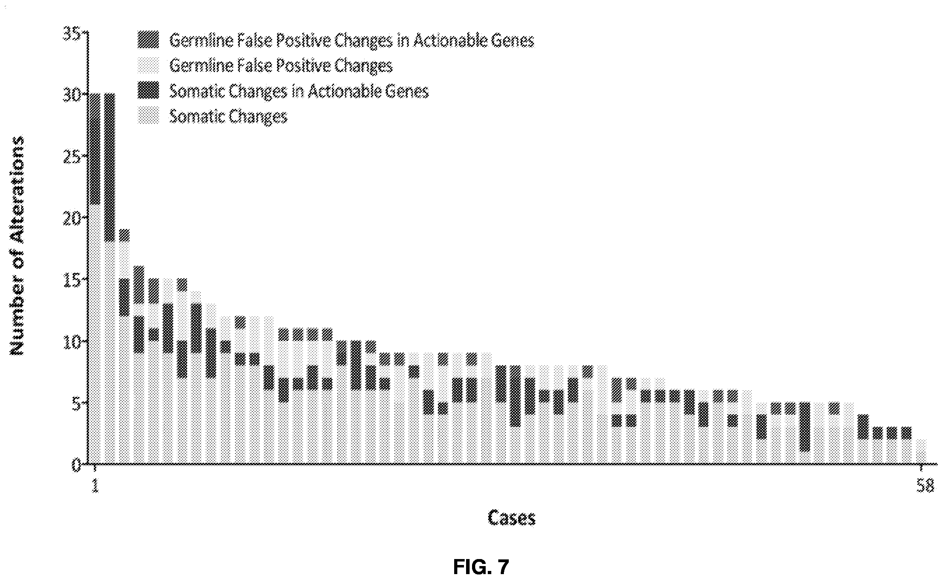

Tumor data from 58 targeted and 100 whole-exome cases were re-analyzed and compared to an unmatched normal sample that had been sequenced using the same methods as for the matched normal samples. Those data were used to remove common germline variants as well as sequencing and alignment errors. All candidate alterations were visually inspected to remove any remaining artifacts. As shown in FIGS. 7-9, an average of 11.53 mutations (range 3 to 34) and 1401 mutations (range 919 to 2651) were observed in the targeted and exome cases, respectively.

FIG. 7 show bar graphs depicting the number of true somatic alterations and germline false positive changes in each case for tumor-only targeted analyses

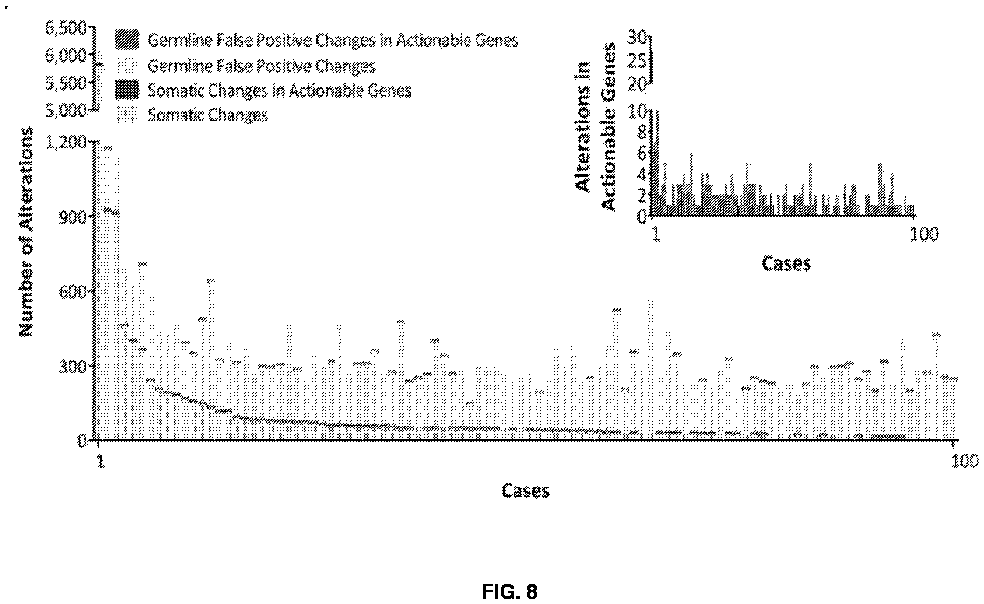

FIG. 8 show bar graphs depicting the number of true somatic alterations and germline false positive changes in each case for exome analyses. In FIGS. 7 and 8, the fraction of changes in actionable genes is indicated for both somatic and germline changes.

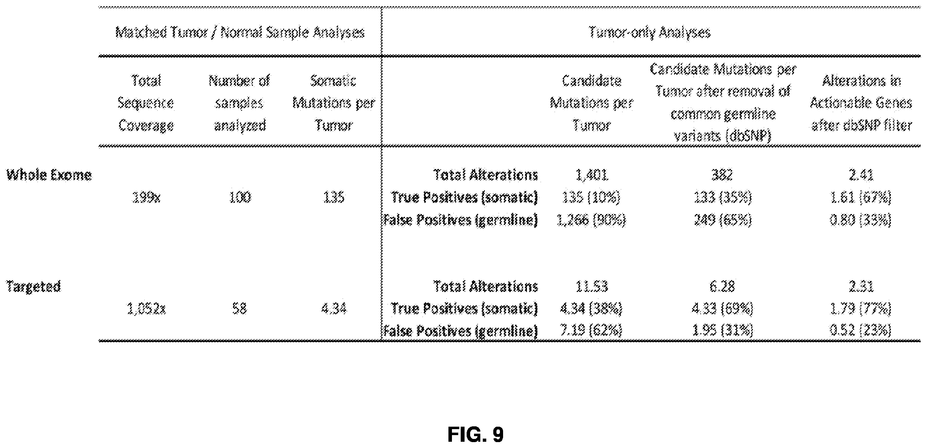

FIG. 9 is a chart summarizing the overall characteristics and the number of somatic and germline variants detected for each type of analysis. For reference, the chart shows total sequence coverage, the number of samples analyzed, and the number of somatic mutations per tumor in the matched tumor/normal analyses.

In order to identify additional germline variants in the tumors that were not present in the unmatched normal, the observed tumor alterations were compared to those in single nucleotide polymorphism (SNP) databases (dbSNP version 138) and filtered variants identified through the 1,000 Genomes Project or other sources (including 42,886,118 total candidate variants). That approach removed between 0 and 9 alterations (average 5.25) in the targeted analyses, including all germline alterations in 10 of 58 cases. However, an average of 1.95 germline variants remained per case through the tumor-only approach, resulting in a total of 113 remaining germline changes in the 58 cases analyzed. A total of 1,019 mutations were removed using dbSNP filters in each of the exome cases (range 623 to 1,911), but an average of 382 mutations remained per case. A significant proportion of the remaining germline variants included changes that could have been classified as potentially actionable changes. For example, a JAK2 mutation in the catalytic domain (1021Y>F), multiple missense alterations in ERBB2, an in-frame deletion (1508PF>P) in TSC2, and an ALK change in the catalytic domain (1200A>V) would have been incorrectly identified through a tumor-only approach. Approved or investigational therapies targeting the altered protein product are available for these genes, including ruxolitinib for JAK2, neratinib for ERBB2, everolimus for TSC2, and crizotinib for ALK, that could have been inappropriately administered to patients based on a tumor-only analysis. Overall, the majority of cases filtered using germline databases had remaining germline alterations, with approximately half in potentially actionable genes.

The filtering of tumor-only data with variants present in germline databases has the potential to inadvertently remove somatic variants that may be identical to germline variants. In the targeted analyses two somatic mutations in PDGFRA (478S>P) and ATRX (929Q>E) matched identical mutations at the nucleotide level in dbSNP and were erroneously removed by that method. The analysis of all coding genes revealed 155 somatic mutations were removed using that approach, including the 114R>C change in the catalytic domain of the mitogen-activated protein kinase MAPK4 and 320P>R in the transcription factor ESX1 which have been previously reported to be somatically mutated in skin, and thyroid and liver cancers, respectively.

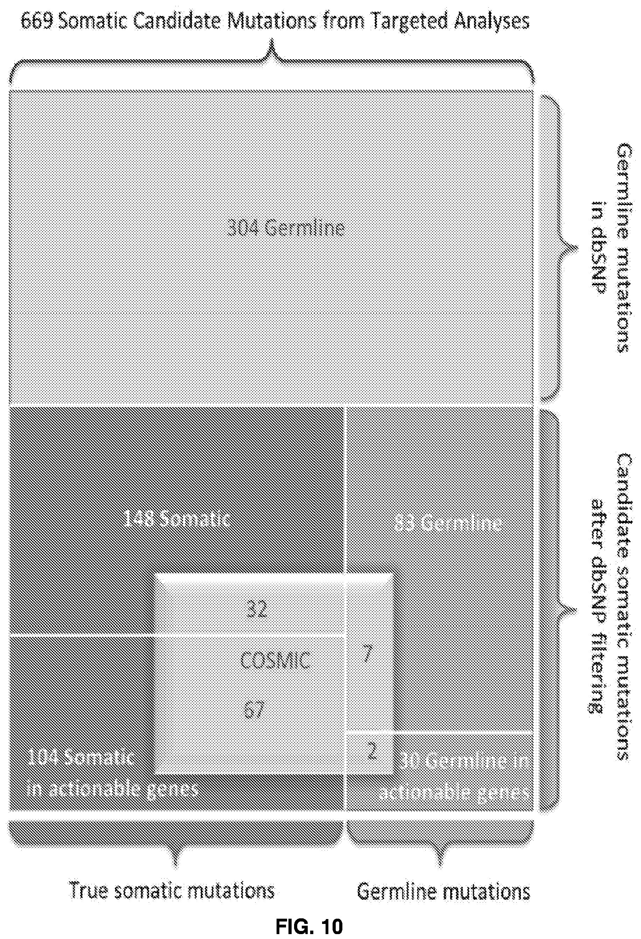

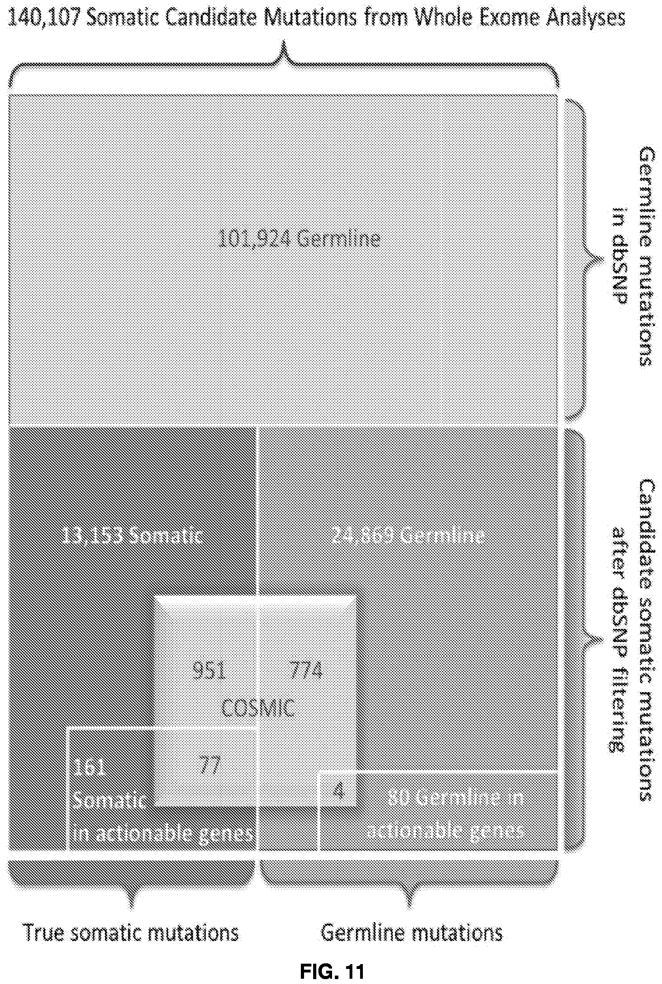

To further examine detection of somatic alterations using a tumor-only approach, the somatic mutations were separated from the remaining germline alterations after dbSNP filtering using data from the COSMIC (Catalogue of Somatic Mutations in Cancer) database. Mutations in the dataset were considered more likely to be somatic if tumor-specific alterations had previously been reported within the same codon of the gene. In total, 108 mutations in 47 of the cases analyzed for the targeted set of genes and 1,806 mutations in the exome cases were classified into this category. That approach was useful in identifying well characterized mutations at hotspots in oncogenes such as KRAS, TP53 and PIK3CA, but did not identify less frequent non-synonymous somatic mutations. Nine of the potential somatic mutations in the targeted genes that overlapped with COSMIC were present in the matched normal samples and were, in fact, germline. In the exome data, 778 germline mutations occurred at codons in which somatic mutations had been previously described. Those bioinformatics filtering approaches are depicted graphically in FIGS. 10 and 11.

FIG. 10 shows how 108 mutations in 47 of the cases analyzed for the targeted set of genes were classified into as somatic and subject to COSMIC filtering.

FIG. 11 how 1,806 mutations in the exome cases were classified as somatic as subject to the COSMIC criteria.

As somatic mutations can be clustered within certain regions of a gene, the COSMIC criteria were expanded to include any mutations within 5 codons of the observed alteration. That increased the number of potential somatic mutations in the targeted genes by 152 to give a total of 270 (4.48 per patient) and increased the number by almost 15,000 in the exome cases to give a total of 16,731 (168 per patient). However, the specificity of the approach was significantly reduced, with 48 and 8,929 of these mutations actually occurring in the matched normal in the targeted and exome genes, respectively. To determine the overall number of identical changes in the genome that had been reported as both germline variants as well as somatic changes through other studies, we examined the overall overlap between common dbSNP variants and the COSMIC databases. After excluding variants of known medical impact or annotated as somatic in dbSNP, 8,606 non-synonymous mutations were present in both databases, of which 63 of these mutations were observed > 5 times in COSMIC. Those analyses suggest that a significant number of variants in the germline may be identical to those in somatic disease such as cancer and the number of identical variants will increase as additional somatic and germline genomes are analyzed.

In some embodiments, quality control techniques include determining a number of false positives by using the methods outlined above, and discussed with respect to FIGS. 7-9. For example, a laboratory, or other test facility, can validate its ongoing rate of false positives by regularly performing the techniques described herein. For example, a tumor sample may be sequenced, and the sequence compared to a library of mutations, such as the COSMIC database. Based upon this comparison, various mutations may be identified in the tumor sample. The mutations identified in the tumor sample, i.e., by comparing to a library, may be compiled in a list of initial actionable mutations. (The list of initial actionable mutations will typically be saved in non-transitory electronic memory, either directly, or as part of a spreadsheet or database.) The list of initial actionable mutations may be compared to the identified tumor-specific mutations, determined using the methods described herein, to assess the quality of the methods that were used to determine the list of initial actionable mutations.

In some embodiments, a user may assign a score to the tumor sample, or the method of evaluating the tumor sample, based upon the similarity between the list of initial actionable mutations and the identified tumor-specific mutations. In some instances, a high score may be assigned to lists of initial actionable mutations that are similar to the identified tumor-specific mutations. In some instances, a low score may be assigned to lists of initial actionable mutations that are similar to the identified tumor-specific mutations. Regardless of whether the score is low or high, the score will reflect the degree of similarity between the list of initial actionable mutations and the identified tumor-specific mutations, with more similarity being indicative of a list of initial actionable mutations that is closer to the "true" result, i.e., mutations that are real and indicative of a real risk of developing a disease, e.g., cancer. Where this score is part of a quality control or quality assurance program, the list of initial actionable mutations may be accepted or rejected based upon the score. In other instances, the list of initial actionable mutations may represent a "test case" for quality control. Thus, if the "test case" has a sufficient score, leading to acceptance of the list of initial actionable mutations, other tumor samples, evaluated in the same way, will be assumed to be of a sufficient quality to be accepted, i.e., reported to a patient, health care provider, hospital, regulatory agency, etc.

In some embodiments, a more detailed analysis of the specificity and sensitivity of the testing performed by the laboratory can be completed by comparing Receiver-Operating Characteristics (ROC) graphs of the lab's techniques, in addition to using the tumor-matched-normal method or the tumor-unmatched-normal method described herein. For additional details of ROC techniques, see, e.g., Zweig, M. H., and Campbell, G., Clin. Chem. 39, 561-577 (1993), incorporated herein by reference in its entirety. An ROC graph is a plot of all of the sensitivity/specificity pairs resulting from continuously varying the decision threshold (disease = yes or no) over the entire range of data observed. An ROC graph depicts the overlap between the two distributions by plotting the sensitivity versus 1-specificity for the complete range of decision thresholds. On the y-axis is sensitivity, or the true-positive fraction [defined as (number of true-positive test results) (number of true-positive+number of false-negative test results]. This has also been referred to as positivity in the presence of a disease or condition. It is calculated solely from the affected subgroup. On the x axis is the false-positive fraction, or 1-specificity [defined as (number of false-positive results)/(number of true-negative+number of false-positive results)]. It is an index of specificity and is calculated entirely from the unaffected subgroup. Using such methods, a user can better evaluate the "true" risk of false positives, because many diseases are influenced by multiple mutations while others are not. In some instances, the risk of misdiagnosis is high because there are only a few mutations associated with the disease or certain mutations are highly correlated with the disease. In other instances, the risk of misdiagnosis is smaller, e.g., because of the disease is correlated with multiple mutations, which must be present for the disease to progress.

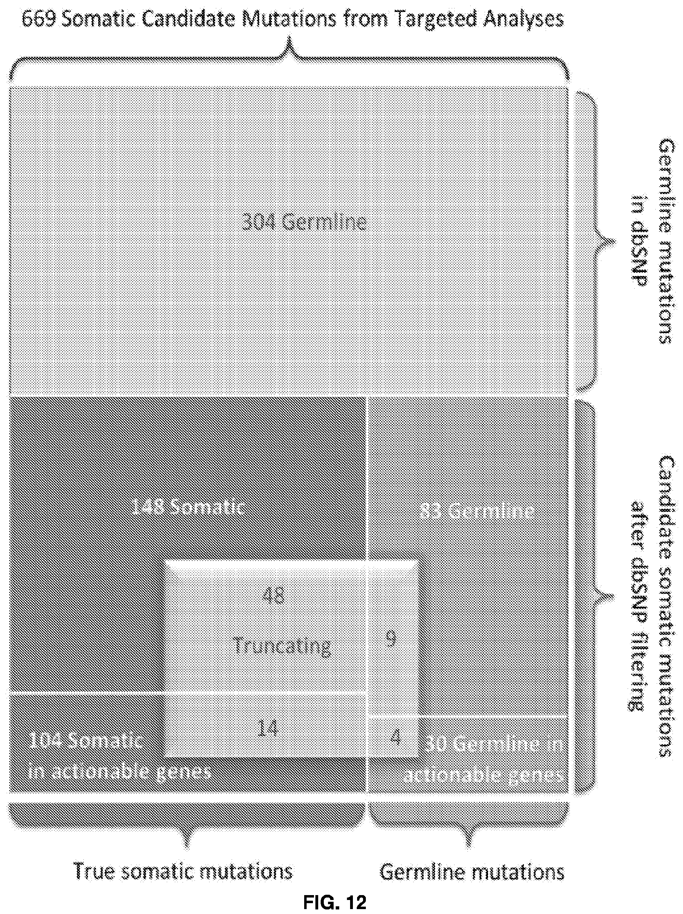

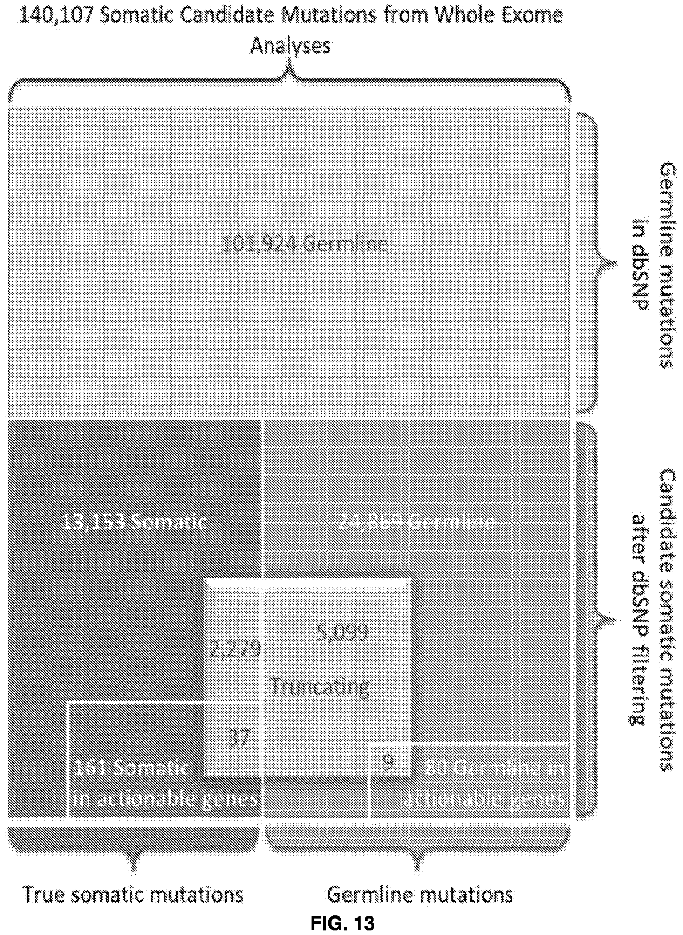

As somatic mutations in tumor suppressor genes are often truncating, that mutation type was also examined as a means to positively select for alterations in the tumor-only data after filtering of common germline variants. Those data are depicted graphically in FIGS. 12 and 13.

FIG. 12 shows the seventy-five mutations in genes such as CDH1 (splice site), PIK3R1 (frameshift) and ARID1B (nonsense) in 43 cases of the targeted analyses that fell into the category of somatic mutations in tumor suppressor genes. Similar to the COSMIC approach, 13 of the alterations identified as candidate somatic changes using that method were germline.

FIG. 13 shows results for the exome cases, with 7,424 truncating mutations, of which 5,108 of these were germline, not somatic.

Additionally, the kinase domain of the protein was searched for mutations, as activating somatic mutations often occur in those regions.

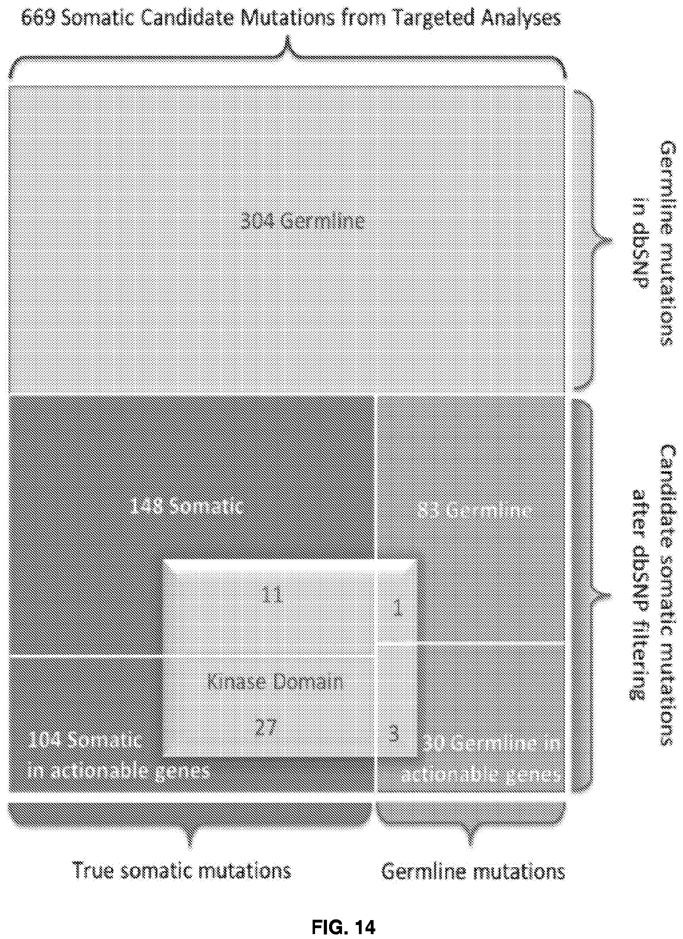

FIG. 14 shows that forty-two alterations, including the EGFR exon 19 deletion 745KELREA>T; 542E>K in PIK3CA; 1021Y>F in JAK2 and 867E>K in RET were identified in the targeted data respectively. Four mutations in the targeted set (including the alteration in JAK2) and 295 alterations in the exome set were in fact germline.

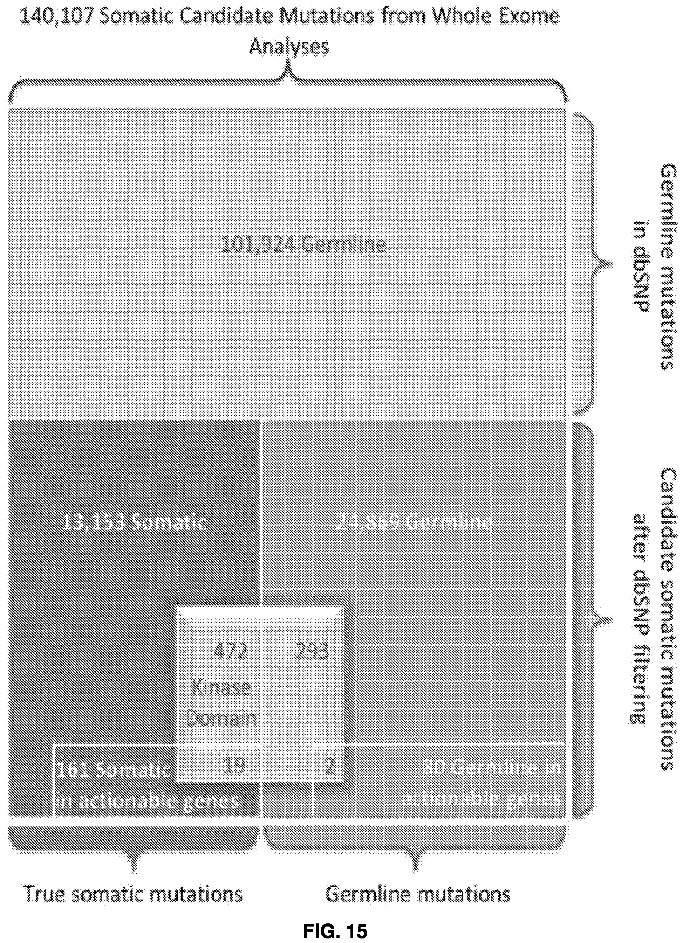

FIG. 15 shows that 786 mutations including 309P>L in MAPK12 and 201P>S in CDK10 were identified in the exome data respectively.

Using a combination of the COSMIC, truncating alteration, and kinase domain approaches, 216 of 252 somatic mutations were correctly identified in the targeted analyses. Of the 36 somatic mutations that were missed, several occurred in genes such as ERBB2, ERBB3 and TSC2 that are under active clinical investigation and may have been clinically actionable. Those approaches also identified 71 mutations (1.22 per case) that were known to be germline from the analyses of the matched normal samples. Those included changes in actionable genes such as ERBB2 (1128V>I), MSH6 (726F>L) and RET (977S>R). Furthermore, there were 78 mutations that were not removed by the SNP filters nor positively selected by the additional criteria and could not be classified by those methods. When the entire coding region was analyzed, only 8,941 of the 13,314 true somatic mutations were identified, 14,734 germline variants were incorrectly categorized as likely to be tumor-specific, and the remaining mutations including 10,135 germline alterations could not be classified.

As an independent measure of the somatic or germline status of a variant, the fraction of mutant alleles in an analyzed tumor sample was examined. Germline mutations would be expected to have variant allele frequency close to 50% for heterozygous and 100% for homozygous changes, whereas the proportion of variant tags for somatic mutations would depend on the level of normal tissue contamination in the tumor sample and would presumably be lower. Of the 43 targeted cases where tumor cellularity was available, only 5 of these had a pathological purity of less than 50%. In those cases all of the alterations were correctly called as somatic or germline using this method. However, in the majority of cases, the tumor cellularity exceeded 50% and this approach could not reliably distinguish between somatic and germline alterations, identifying on average only 48% of somatic mutations correctly. Likewise, although twenty of the likely cancer predisposing changes that were identified could be detected in germline databases, only two of the assessable 16 germline variants could be distinguished from somatic alterations through an analysis of allele fractions.

### Incorporation by Reference

References and citations to other documents, such as patents, patent applications, patent publications, journals, books, papers, web contents, have been made throughout this disclosure. All such documents are hereby incorporated herein by reference in their entirety for all purposes.

### Equivalents

Various modifications of the invention and many further embodiments thereof, in addition to those shown and described herein, will become apparent to those skilled in the art from the full contents of this document, including references to the scientific and patent literature cited herein. The subject matter herein contains important information, exemplification and guidance that can be adapted to the practice of this invention in its various embodiments and equivalents thereof.
The following paragraphs, describing aspects of the invention, are part of the description:
1. A method of analyzing a nucleic acid for a biomarker associated with a tumor, the method comprising:
   providing a tumor sequence read generated by sequencing nucleic acid from a tumor sample of a patient;
   providing a normal sequence read generated by sequencing nucleic acid from a normal sample of the patient;
   comparing the tumor and normal sequence reads;
   filtering the tumor sequence reads based on the comparison; and
   identifying a tumor-specific mutation in the filtered sequence reads.
2. The method of para. 1, wherein the method further comprises, prior to the providing steps, sequencing the nucleic acid from the tumor sample and the normal sample.
3. The method of para. 1, wherein the comparing step comprises aligning the tumor and normal sequence reads to a reference, and comparing the aligned normal reads to the aligned tumor reads.
4. The method of para. 1, wherein the comparing step comprises creating a tumor contig based on the tumor sequence reads and a normal contig based on the normal sequence reads, and comparing the normal contig to the tumor contig.
5. The method of para. 1, further comprising comparing the filtered sequence reads to a reference.
6. The method of para. 5, wherein the reference is selected from a group consisting of a tumor reference and a normal reference.
7. The method of para. 1, further comprising comparing the sequence reads to a Sanger sequence reference.
8. The method of para. 1, wherein the tumor and normal sequence reads correspond to a panel of genes known to be associated with cancer.
9. The method of para. 1, wherein the tumor and normal sequence reads correspond to coding regions.
10. The method of para. 1, wherein the tumor and normal sequence reads correspond to intronic regions.
11. The method of para. 1, wherein filtering comprises excluding loci that do not meet a threshold.
12. The method of para. 1, wherein the tumor sample comprises a biopsy specimen and circulating tumor DNA.
13. The method of para. 1, wherein the normal sample is selected from the group consisting of lymphocytes, a saliva sample, and a buccal sample.
14. The method of para. 1, further comprising determining a prognosis for the patient based on the biomarker.
15. The method of para. 1, further comprising designing a treatment regimen for the patient based on the biomarker.
16. The method of para. 1, further comprising comparing the tumor sequence read to a library of mutations to determine a list of initial actionable mutations; and
   comparing the identified tumor-specific mutations to the list of initial actionable mutations.
17. The method of para. 16, further comprising assigning a score to the tumor sequence based upon the comparison between the identified tumor-specific mutations and the list of initial actionable mutations.
18. The method of para. 17, further comprising accepting the list of initial actionable mutations based upon the score.
19. A system for identifying a mutation in a nucleic acid, the system comprising a processor coupled to a non-transitory memory containing instructions executable by the processor to cause the system to:
   receive a tumor sequence read generated by sequencing nucleic acid from a tumor sample of a patient;
   receive a normal sequence read generated by sequencing nucleic acid from a normal sample of the patient;
   compare the tumor and normal sequence reads;
   filter the tumor sequence reads based on the comparison; and
   assess the filtered sequence reads for a mutation, wherein the mutation is a biomarker associated with the tumor.
20. The system of para. 19, further comprising a nucleic acid sequencing instrument operable to sequence the nucleic acid from the tumor sample and the normal sample.
21. The system of para. 19, wherein the system is further operable to align the tumor and normal sequence reads to a reference, and compare the aligned normal reads to the aligned tumor reads.
22. The system of para. 19, wherein the system is further operable to create a tumor contig based on the tumor sequence reads and a normal contig based on the normal sequence reads, and compare the normal contig to the tumor contig.
23. The system of para. 19, wherein the system is further operable to compare the filtered sequence reads to a reference.
24. The system of para. 23, wherein the reference is selected from a group consisting of a tumor reference and a normal reference.
25. The system of para. 19, wherein the system is further operable to compare the sequence reads to a Sanger sequence reference.
26. The system of para. 19, wherein the tumor and normal sequence reads correspond to a panel of genes known to be associated with cancer.
27. The system of para. 19, wherein the tumor and normal sequence reads correspond to coding regions.
28. The system of para. 19, wherein the tumor and normal sequence reads correspond to intronic regions.
29. The system of para. 19, wherein the system is further operable to filter by excluding loci that do not meet a threshold.
30. The system of para. 19, wherein the tumor sample comprises a biopsy specimen and circulating tumor DNA.
31. The system of para. 19, wherein the normal sample is selected from the group consisting of lymphocytes, a saliva sample, and a buccal sample.
32. The system of para. 19, wherein the system is further operable to determine a prognosis for the patient based on the biomarker.
33. The system of para. 19, wherein the system is further operable to design a treatment regimen for the patient based on the biomarker.
34. A method of analyzing a nucleic acid for variations associated with a tumor, the method comprising:
   sequencing nucleic acid obtained from a tumor sample, thereby generating tumor sequence reads;
   sequencing nucleic acid obtained from a normal sample, thereby generating normal sequence reads;
   aligning the tumor and normal sequence reads to a reference sequence;
   identifying one or more variations in the tumor and normal sequence reads based on the alignment; and
   filtering, based on a threshold, variations that differ between the tumor and normal sequence reads, wherein variations above the threshold are biomarkers associated with the tumor.
35. The method of para. 34, further comprising comparing the filtered variations to a reference.
36. The method of para. 35, wherein the reference is selected from a group consisting of a tumor reference and a normal reference.
37. The method of para. 34, further comprising comparing the sequence reads to a Sanger sequence reference.
38. The method of para. 34, wherein the tumor and normal sequence reads correspond to a panel of genes known to be associated with cancer.
39. The method of para. 34, wherein the tumor and normal sequence reads correspond to coding regions.
40. The method of para. 34, wherein the tumor and normal sequence reads correspond to intronic regions.
41. The method of para. 34, wherein the tumor sample comprises a biopsy specimen and circulating tumor DNA.
42. The method of para. 34, wherein the normal sample is selected from the group consisting of lymphocytes, a saliva sample, and a buccal sample.
43. The method of para. 34, further comprising determining a prognosis for the patient based on the biomarker.
44. The method of para. 34, further comprising designing a treatment regimen for the patient based on the biomarker.
45. The method of para. 34, further comprising comparing the tumor sequence read to a library of mutations to determine a list of initial actionable mutations; and
   comparing the identified tumor-specific mutations to the list of initial actionable mutations.
46. The method of para. 45, further comprising assigning a score to the tumor sequence based upon the comparison between the identified tumor-specific mutations and the list of initial actionable mutations.
47. The method of para. 46, further comprising accepting the list of initial actionable mutations based upon the score.
48. A method for reducing a rate of false positives mutation calls when sequencing a tumor sample, the method comprising:
   providing a tumor sequence read generated by sequencing nucleic acid from a tumor sample of a patient;
   providing a normal sequence read generated by sequencing nucleic acid from a normal sample of the patient;
   comparing the tumor and normal sequence reads;
   filtering the tumor sequence reads based on the comparison; and
   identifying a tumor-specific mutation in the filtered sequence reads.
49. A method for reducing a rate of false positives mutation calls when sequencing a tumor sample, the method comprising:
   sequencing nucleic acid obtained from a tumor sample, thereby generating tumor sequence reads;
   sequencing nucleic acid obtained from a normal sample, thereby generating normal sequence reads;
   aligning the tumor and normal sequence reads to a reference sequence;
   identifying one or more variations in the tumor and normal sequence reads based on the alignment; and
   filtering, based on a threshold, variations that differ between the tumor and normal sequence reads, wherein variations above the threshold are biomarkers associated with the tumor.
50. A method for verifying the quality of a tumor sequencing technique, the method comprising:
   providing a tumor sequence read generated by sequencing nucleic acid from a tumor sample of a patient with a sequencing technique;
   providing a normal sequence read generated by sequencing nucleic acid from a normal sample of the patient;
   comparing the tumor sequence read to a library of mutations to determine a list of initial actionable mutations;
   comparing the tumor and normal sequence reads;
   filtering the tumor sequence reads based on the comparison;
   identifying a tumor-specific mutation in the filtered sequence reads; and
   comparing the identified tumor-specific mutations to the list of initial actionable mutations.
51. The method of para. 50, further comprising assigning a score to the tumor sequencing technique based upon the comparison between the identified tumor-specific mutations and the list of initial actionable mutations.
52. The method of para. 51, further comprising accepting the list of initial actionable mutations based upon the score.
53. A method for verifying the quality of a tumor sequencing technique, the method comprising:
   sequencing nucleic acid obtained from a tumor sample using a technique, thereby generating tumor sequence reads;
   sequencing nucleic acid obtained from a normal sample, thereby generating normal sequence reads;
   aligning the tumor and normal sequence reads to a reference sequence;
   identifying one or more variations in the tumor and normal sequence reads based on the alignment;
   filtering, based on a threshold, variations that differ between the tumor and normal sequence reads, wherein variations above the threshold are biomarkers associated with the tumor, thereby producing a compilation of tumor-specific mutations;
   comparing the tumor sequence read to a library of mutations to determine a list of initial actionable mutations; and
   comparing the identified tumor-specific mutations to the list of initial actionable mutations.
54. The method of para. 53, further comprising assigning a score to the tumor sequencing technique based upon the comparison between the identified tumor-specific mutations and the list of initial actionable mutations.
55. The method of para. 54, further comprising accepting the list of initial actionable mutations based upon the score.

## Claims

1. A computer-implemented method, comprising:
obtaining tumor sequencing data from a tumor sample obtained from a subject;
obtaining normal sequencing data from a normal sample obtained from the subject;
identifying tumor specific variants by comparing the tumor sequencing data to the normal sequencing data, wherein each tumor specific variant is a single base substitution, an insertion, or a deletion;
identifying candidate somatic mutations from the tumor specific variants based on one or more predetermined criteria using the tumor sequencing data and the normal sequencing data;
identifying protein-coding mutations from the candidate somatic mutations based on gene annotation, wherein the protein-coding mutations occur in protein-coding regions;
identifying protein-altering mutations from the protein-coding mutations based on predicted functional consequences of the protein-coding mutations; and
assessing the protein-altering mutations to identify clinically actionable genes using one or more therapies.

2. The computer-implemented method of claim 1, further comprising:
sequencing nucleic acid obtained from the tumor sample to generate the tumor sequencing data, wherein the tumor sequencing data comprises tumor sequence reads and corresponding quality scores;
sequencing nucleic acid obtained from the normal sample to generate the normal sequencing data, wherein the normal sequencing data comprises normal sequence reads and corresponding quality scores; and
filtering the tumor sequencing data and the normal sequencing data before the identifying the tumor specific variants based on the corresponding quality scores.

3. The computer-implemented method of claim 2, further comprising:
identifying a position of each of the tumor sequence reads by aligning the tumor sequence reads to a human reference genome;
identifying a position of each of the normal sequence reads by aligning the normal sequence reads to the human reference genome;
identifying the most prevalent nucleotides of the filtered tumor sequence reads at each position to generate one or more tumor consensus sequences; and
identifying the most prevalent nucleotides of the filtered normal sequence reads at each position to generate one or more normal consensus sequences;
wherein the tumor specific variants are identified by comparing the tumor consensus sequences to the normal consensus sequences.

4. The computer-implemented method of claim 2 or 3, wherein the sequencing is targeted sequencing.

5. The computer-implemented method of any of claims 1-4, wherein a tumor specific variant is identified as a candidate somatic mutation when:
(i) the tumor specific variant is contained in distinct tumor sequence reads;
(ii) a number of distinct tumor sequence reads containing the tumor specific variant meets a percentage threshold;
(iii) the tumor specific variant is contained in at most a predetermined percent of distinct normal sequence reads;
(iv) the tumor specific variant is not present in a custom database of germline variants;
(v) a location of the tumor specific variant is covered by both the tumor sequencing data and the normal sequencing data; and/or
(vi) the tumor specific variant is not a result of misplaced alignment, wherein the misplaced alignment is determined by searching a reference genome.

6. The computer-implemented method of any of claims 1-5, further comprising predicting functional consequences of the protein-coding mutations using an effect prediction tool and a custom database comprising gene and clinical annotations.

7. The computer-implemented method of any of claims 1-6, wherein a gene is clinically actionable when the gene is associated with: (i) a known approved therapy, (ii) a therapy in a published prospective or retrospective clinical study, or (iii) an existing clinical trial or study, based on gene and clinical annotations.

8. The computer-implemented method of any of claims 1-7, wherein the subject is a patient having been diagnosed of a cancer.

9. The computer-implemented method of claim 8, wherein the cancer is selected from breast, skin, colorectal, pancreatic, ovarian, prostate, or cervical brain, cholangiocarcinomas, head and neck, neuroendocrine, renal, gastric, gynecological, esophageal, melanoma, hematopoietic malignancies, and sarcomas.

10. The computer-implemented method of any of claims 1-9, wherein the protein-altering mutations are identified by analyzing truncating alterations in cancer-related genes from the protein-coding mutations, wherein the truncating alterations comprises frameshift mutations, splice site changes, and nonsense mutations.

11. The computer-implemented method of claim 10, wherein the cancer-related genes are selected from the group consisting of: ALK; APC; ATM; AXIN2; BAP1; BLM; BMPR1A; BRCA1; BRCA2; BRIP1; BUB1B; CDC73; CDH1; CDK4; CDKN2A; CHEK2; CREBBP; CYLD; DDB2; DICER1; EP300; ERCC2; ERCC3; ERCC4; ERCC5; EXT1; EXT2; FANCA; FANCB; FANCC; FANCD2; FANCE; FANCF; FANCG; FANCI; FANCL; FANCM; FH; FLCN; GPC3; KIT; MEN1; MET; MLH1; MSH2; MSH6; MUTYH; NBN; NF1; NF2; PALB2; PDGFRA; PHOX2B; PMS2; POLD1; POLE; POLH; POT1; PRKAR1A; PRSS1; PTCH1; PTEN; RAD51C; RB1; RECQL4; RET; SBDS; SDHAF2; SDHB; SDHC; SDHD; SMAD4; STK11; SUFU; TERT; TP53; TSC1; TSC2; VHL; WAS; WRN; WT1; XPA; and XPC.

12. The computer-implemented method of any of claims 1-11, wherein the tumor sample comprises circulating tumor DNA (ctDNA) isolated from a blood sample of the subject, and wherein the normal sample is a tumor-free sample.

13. The computer-implemented method of any of claims 1-12, further comprising
developing an individualized prognosis and treatment regimen for the subject based on the clinically actionable genes specific to the subject.

14. A system, comprising:
one or more processors; and
one or more non-transitory memories comprising computer program instructions that when executed by the one or more processors, cause the one or more processors to perform operations in any of claims 1-13.

15. A non-transitory machine-readable medium comprising computer program instruction that, when executed by one or more processors, cause the one or more processors to perform operations in any of claims 1-13.
